# EUROPÄISCHE PATENTSCHRIFT

(11) **EP 1 913 038 B1**
(45) Veröffentlichungstag und Bekanntmachung des Hinweises auf die Patenterteilung: **27.03.2013**
(21) Anmeldenummer: 06764242.1
(22) Anmeldetag: 21.07.2006
(51) Int. Cl.: C08F 220/04

(54) **AMPHOLYTISCHES COPOLYMER, DESSEN HERSTELLUNG UND VERWENDUNG**
AMPHOLYTIC COPOLYMER, PRODUCTION THEREOF, AND USE OF THE SAME
COPOLYMERE AMPHOLYTE, SA PRODUCTION ET SON UTILISATION

(30) Priorität: 22.07.2005 DE 102005034412
(43) Veröffentlichungstag der Anmeldung: 23.04.2008
(73) Patentinhaber: BASF SE, 67056 Ludwigshafen (DE)
(72) Erfinder: NGUYEN-KIM, Son, 69502 Hemsbach (DE); GARCIA CASTRO, Ivette, 67067 Ludwigshafen (DE); MATHAUER, Klemens, 69115 Heidelberg (DE); WENDEL, Volker, 64342 Seeheim-Jugenheim (DE); VÖLLMAR, Helmuth, 67061 Ludwigshafen (DE); KAISER, Thomas, 67454 Hassloch (DE)
(86) Internationale Anmeldenummer: PCT/EP2006/064504
(87) Internationale Veröffentlichungsnummer: WO 2007/010034

(56) Entgegenhaltungen:
- WO-A-00/39176
- WO-A-01/62809
- WO-A-93/22358
- WO-A2-2004/058837

## Beschreibung

Die vorliegende Erfindung betrifft ein ampholytisches Copolymer, ein Verfahren zu dessen Herstellung, kosmetische oder pharmazeutische Mittel, die wenigstens ein solches ampholytisches Copolymer enthalten sowie weitere Verwendungen dieser Copolymere.

Polymere mit einer größeren Anzahl ionisch dissoziierbarer Gruppen in der Hauptkette und/oder einer Seitenkette werden als Polyelektrolyte bezeichnet. Weisen diese Polymere sowohl anionogene/anionische als auch kationogene/kationische Gruppen auf, so handelt es sich um amphotere Polyelektrolyte bzw. ampholytische Polymere. Ampholytische Polymere mit ausreichender Anzahl dissoziierbarer Gruppen sind wasserlöslich oder wasserdispergierbar und haben vielfältige Anwendungen im Bereich der Anstrichmittel, Papierhilfsmittel, Hygienemittel, bei der Textilherstellung sowie speziell in der Pharmazie und Kosmetik gefunden.

Kosmetisch und pharmazeutisch akzeptable wasserlösliche Polymere dienen beispielsweise in Seifen, Cremes und Lotionen als Formulierungsmittel, z. B. als Verdicker, Schaumstabilisator oder Wasserabsorbens oder auch dazu, die reizende Wirkung anderer Inhaltsstoffe abzumildern oder die dermale Applikation von Wirkstoffen zu verbessern. Ihre Aufgabe in der Haarkosmetik besteht darin, die Eigenschaften des Haares zu beeinflussen. In der Pharmazie dienen sie beispielsweise als Beschichtungsmittel oder Bindemittel für feste Arzneiformen. Für die Haarkosmetik werden filmbildende Polymere mit ionischen Gruppen beispielsweise als Conditioner dazu eingesetzt, um die Trocken- und Nasskämmbarkeit, das Anfassgefühl, den Glanz und die Erscheinungsform des Haars zu verbessern sowie dem Haar antistatische Eigenschaften zu verleihen. Je nach Anwendungszweck werden dabei wasserlösliche Polymere mit kationischen oder anionischen Funktionalitäten eingesetzt. So weisen Polymere mit kationischen funktionellen Gruppen strukturell bedingt eine hohe Affinität zur negativ geladenen Oberfläche des Haares auf. Polymere mit anionischen Funktionalitäten, wie z. B. gegebenenfalls vernetzte Polyacrylsäure, dienen beispielsweise als Verdicker, weiterhin werden Carboxylatgruppen-haltige Polymere beispielsweise zur Festigung von Haarfrisuren eingesetzt.

Schwierigkeiten bereitet oft die Bereitstellung von Produkten mit einem komplexen Eigenschaftsprofil. So besteht ein Bedarf an Polymeren für kosmetische und andere Mittel, die über gute Filmbildungseigenschaften verfügen und gleichzeitig eine Einstellung der rheologischen Eigenschaften der Mittel ermöglichen. Zudem werden an kosmetische und pharmazeutische Produkte vom Verbraucher zunehmend ästhetische Anforderungen gestellt. So wird bei derartigen Produkten derzeit eine Bevorzugung von klaren, opaquen Formulierungen in Form von Gelen beobachtet. In vielen Fällen lässt sich das gewünschte Eigenschaftsprofil nur durch Einsatz mehrerer Polymere mit ionischen Gruppen erzielen. Dabei zeigt sich jedoch häufig eine Unverträglichkeit der verschiedenen Polymere miteinander, was beispielsweise zu einem unerwünschten Aussalzen führen kann. Es besteht daher Bedarf an kosmetisch und pharmazeutisch verträglichen ampholytischen Polymeren, die bei einem Einsatz als einzige Polymerkomponente geeignet sind, ein bestimmtes Eigenschaftsprofil bezüglich der Filmbildung und der Rheologie bereitzustellen und/oder die mit einer Vielzahl verschiedener Polyelektrolyte gut verträglich sind.

Des Weiteren werden Wirkstoffe für die Kosmetik, aber auch für Arzneimittel, Hygieneprodukte, den Materialschutz etc., d. h. Substanzen, die auch in geringer Konzentration bereits eine Wirkung entfalten, z. B. eine kosmetische Wirkung auf der Haut oder den Haaren, pharmakologische Wirkung in einem Organismus etc., häufig in Form wässriger Wirkstoffzubereitungen formuliert und angewendet. Alternativ ist auch eine Formulierung und Verabreichung in fester Form, z. B. als Pulver oder Pressling (Tablette, etc.) möglich, wobei der Transport an den eigentlichen Wirkort jedoch die Umwandlung in eine wässrige Form umfasst. Ein prinzipielles Problem bei wässrigen Wirkstoffzubereitungen ist die geringe Wasserlöslichkeit vieler Wirkstoffe, die häufig weniger als 5 g/l bei 23 °C/1013 mbar beträgt. Auch hier besteht ein ständiger Bedarf an kosmetisch und pharmazeutisch verträglichen ampholytischen Polymeren, die gleichzeitig als Solubilisatoren für nicht oder nur wenig wasserlösliche Komponenten dienen.

Die US 3,915,921 beschreibt Copolymere, die eine olefinisch ungesättigte Carbonsäure, ein C₁₀-C₃₀(Alkyl(meth)acrylat und gegebenenfalls ein vernetzendes Monomer mit wenigstens 2 ethylenisch ungesättigten Doppelbindungen einpolymerisiert enthalten. In neutralisierter Form dienen sie als Verdicker für diverse Anwendungen.

Die WO 97/21744 beschreibt vernetzte anionische Copolymere und deren Verwendung als Verdickungs- und Dispergiermittel in wässrigen Systemen.

Die EP-A-0 982 021 beschreibt die Verwendung von (teil)neutralisierten Copolymerisaten aus
A) 50 bis 99 Gew.-% monoethylenisch ungesättigten Carbonsäuren und
B) 1 bis 50 Gew.-% mindestens eines Comonomers, ausgewählt unter
   a) monoethylenisch ungesättigten Carbonsäureestern mit gesättigten C₈-C₃₀-Alkoholen,
   b) N-C₈-C₁₈-Alkyl- und N,N-Di-C₈-C₁₈-alkylcarbonsäureamiden,
   c) Vinylestem aliphatischer C₈-C₃₀-Carbonsäuren.
   d) C₈-C₁₈-Alkylvinylethern,
   und Mischungen davon
als Verdicker zur Herstellung von Haarwaschmitteln.
Die WO 01/62809 beschreibt ein kosmetisches Mittel, das wenigstens ein wasserlösliches oder wasserdispergierbares Polymer-enthält, das
a) 5 bis 50 Gew.-% wenigstens eines α,β-ethylenisch ungesättigten Monomers mit einer tert.-Butylgruppe,
b) 25 bis 90 Gew.-% wenigstens eines N-Vinylamids und/oder N-Vinyllactams,
c) 0,5 bis 30 Gew.-% wenigstens einer Verbindung mit einer radikalisch polymerisierbaren, α,β-ethylenisch ungesättigten Doppelbindung und mindestens einer kationogenen und/oder kationischen Gruppe pro Molekül, und
d) 0 bis 30 Gew.-% wenigstens einer weiteren α,β-ethylenisch ungesättigten Verbindung, wobei es sich um Verbindungen mit mindestens einer anionogenen und/oder anionischen Gruppe pro Molekül handeln kann,
eingebaut enthält.

Die EP-A-670 333 beschreibt vernetzte wasserlösliche Polymerdispersionen, die durch Polymerisation eines Monomergemischs, enthaltend wenigstens ein wasserlösliches Monomer, wenigstens einen Vernetzer sowie gegebenenfalls hydrophobe und/oder amphiphile Monomere in Gegenwart eines polymeren Dispergiermittels erhältlich sind. Als wasserlösliche Monomere können neben einer Vielzahl weiterer auch N-Vinylpyrrolidon sowie Monomere mit kationischen/kationisierbaren Gruppen, wie N-Vinylimidazol, eingesetzt werden. Bei den polymeren Dispergiermitteln kann es sich um Polyelektrolyte handeln, die beispielsweise Salze der (Meth)acrylsäure als anionische Monomerbausteine oder quaternierte Derivate von N,N-Dimethylaminoethyl-(meth)acrylat als kationische Bausteine einpolymerisiert enthalten. Ein Einsatz der Polymerdispersionen in der Kosmetik wird nicht beschrieben.

Die EP-A-929 285 lehrt die Verwendung von wasserlöslichen Copolymeren, die Vinylcarbonsäureamid-Einheiten und Vinylimidazol-Einheiten einpolymerisiert enthalten als Bestandteil kosmetischer Mittel. Polyelektrolyt-Komplexe dieser Copolymere mit Carbonsäuregruppen-haltigen Polymeren sind in diesem Dokument nicht offenbart.

Die WO 00/27893 beschreibt wässrige Polymerdispersionen auf der Basis von N-Vinylcarbonsäureamiden und gegebenenfalls Comonomeren, wobei neben einer großen Anzahl weiterer auch N-Vinylpyrrolidon, N-Vinylimidazol und N-Vinylimidazolderivate genannt werden. Die Polymerisation erfolgt in Gegenwart wenigstens eines polymeren Dispergiermittels. Ein Einsatz dieser Polymerdispersionen in der Kosmetik wird nur ganz allgemein und ohne Beleg durch ein Ausführungsbeispiel beschrieben.

Die EP-A-1038891 beschreibt wasserlösliche oder wasserdispergierbare polymere Salze aus wenigstens einem Polymer und wenigstens einem entgegengesetzt geladenen Neutralisationsmittel.

Die WO 02/41856 beschreibt die Verwendung von Polymerdispersionen, die durch Polymerisation wenigstens eines wasserlöslichen Monomers in einer wässrigen Salzlösung, die wenigstens einen Polyelektrolyten als Dispergiermittel enthält, erhältlich sind zur kosmetischen Behandlung von keratinischen Materialien. Zusätzlich enthalten die Dispersionen wenigstens ein Mittel zur Einstellung der Viskosität, beispielsweise eine Polycarbonsäure oder ein Salz davon. Als wasserlösliche Monomere können kationische, anionische und nichtionische Monomere eingesetzt werden, bevorzugt sind Monomergemische, die wenigstens ein kationisches Monomer sowie gegebenenfalls zusätzlich Acrylsäure und/oder Acrylamid enthalten.

Die WO 2005/004821 beschreibt ein kosmetisches oder pharmazeutisches Mittel, das wenigstens einen Polyelektrolyt-Komplex enthält, der wenigstens ein wasserlösliches oder wasserdispergierbares Copolymer A1) mit kationogenen Gruppen, das Vinylimidazol und/oder ein Derivat davon und wenigstens ein weiteres damit copolymerisierbares Monomer einpolymerisiert enthält, und wenigstens ein Säuregruppen-haltiges Polymer A2) umfasst.

Die WO 2005/005497 beschreibt eine wässrige Polymerdispersion Pd), die erhältlich ist durch radikalische Polymerisation eines Monomergemischs M), enthaltend
a) wenigstens eine α,β-ethylenisch ungesättigte amidgruppenhaltige Verbindung der allgemeinen Formel **I** 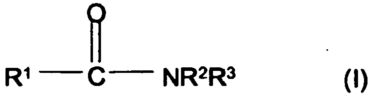 wobei
   R² für eine Gruppe der Formel CH₂=CR⁴-steht und R¹ und R³ unabhängig voneinander für H, Alkyl, Cycloalkyl, Heterocycloalkyl, Aryl oder Hetaryl stehen, oder R¹ und R³ gemeinsam mit der Amidgruppe, an die sie gebunden sind, für ein Lactam mit 5 bis 8 Ringatomen stehen,
b) wenigstens eine radikalisch polymerisierbare vernetzende Verbindung mit wenigstens zwei α,β-ethylenisch ungesättigten Doppelbindungen pro Molekül,
c) wenigstens eine Verbindung mit einer radikalisch polymerisierbaren α,β-ethylenisch ungesättigten Doppelbindung und mindestens einer kationogenen und/oder kationischen Gruppe pro Molekül,

in einem wässrigen Medium in Gegenwart wenigstens eines polymeren anionischen Dispergiermittels D). Beschrieben sind weiterhin kosmetische oder pharmazeutische Mittel, die eine solche Polymerdispersion oder ein durch Trocknen einer solchen Dispersion erhältliches Polymer enthalten.

Die WO 00/39176 beschreibt ein hydrophiles, kationisches, ampholytisches Copolymer, das 0,05 bis 20 Mol-% eines anionischen Monomers mit wenigstens einer Carboxygruppe, 0 bis 45 Mol-% eines kationischen Monomers mit wenigstens einer Aminogruppe sowie gegebenenfalls ein hydrophobes Monomer und/oder einen Vernetzer einpolymerisiert enthält, wobei das Molverhältnis von kationischem zu anionischem Monomer etwa 2 : 1 bis 16 : 1 beträgt. Diese ampholytischen Copolymere können unter Anderem zur Modifizierung rheologischer Eigenschaften von Körperpflegemitteln eingesetzt werden.

Die WO 04/058837 beschreibt ein ampholytisches Copolymer, das durch radikalische Copolymerisation von
a) wenigstens einer ethylenisch ungesättigten Verbindung mit mindestens einer anionogenen und/oder anionischen Gruppe,
b) wenigstens einer ethylenisch ungesättigten Verbindung mit mindestens einer kationogenen und/oder kationischen Gruppe,
c) wenigstens einer ungesättigten amidgruppenhaltigen Verbindung sowie gegebenenfalls weiterer Comonomere erhältlich ist. Beschrieben sind weiterhin Polyelektrolyt-Komplexe, die ein solches ampholytisches Copolymer enthalten sowie kosmetisch oder pharmazeutische Mittel auf Basis dieser ampholytischen Copolymere und Polyelektrolyt-Komplexe.

Die US 2006/0084586 A1 beschreibt Rheologie-modifizierende Haarfestigerharze, die ein vernetztes Copolymer auf Basis von Vinylamid- und Carbonsäuremonomeren enthalten. Polymere, die wenigstens eine kationische Verbindung, die ausgewählt ist unter N-Vinylimidazol-Verbindungen, N-[3-(dimethylamino)propyl]acrylamid, N-(3-(dimethylamino)propyl]methacrylamid und Mischungen davon, einpolymerisiert enthalten, sind nicht beschrieben.

Die WO 93/22358 beschreibt Copolymere aus Carbonsäuren und quartären Ammoniumverbindungen und ihre Verwendung als Verdickungs- oder Dispergiermittel.

Es besteht nach wie vor Bedarf an Polymeren die neben guten filmbildenden Eigenschaften auch eine Einstellung der rheologischen Eigenschaften der Produkte zulassen, so dass sie z. B. in Form von Mousses oder Gelen formuliert werden können. Ferner sind Polymere wünschenswert, die sich als Solubilisatoren für nicht oder nur wenig wasserlösliche Komponenten eignen und/oder sich durch eine gute Verträglichkeit mit anderen Formulierungsbestandteilen auszeichnen. Polymere für einen Einsatz in der Haarkosmetik sollen sich zudem durch weitere vorteilhafte Eigenschaften, wie z. B. die Erzeugung elastischer Frisuren bei gleichzeitig starker Festigung auch bei hoher Luftfeuchtigkeit, gute Auswaschbarkeit und durch guten Griff des damit behandelten Haares auszeichnen.

Ein erster Gegenstand der Erfindung ist ein ampholytisches Copolymer A), erhältlich durch radikalische Copolymerisation nach der Methode der Fällungspolymerisation, das Verbindungen, bestehend aus
a) 2 bis 96 Gew.-% Acrylsäure und/oder Methacrylsäure,
b) 2 bis 96 Gew.-% wenigstens einer N-Vinylimidazol-Verbindung der allgemeinen Formel (II) 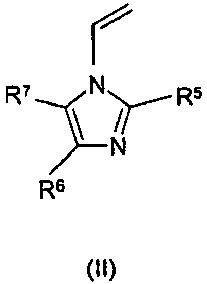 worin R⁵ bis R⁷ unabhängig voneinander für Wasserstoff, C₁-C₄-Alkyl oder Phenyl stehen, oder die durch Protonierung oder Quaternisierung der N-Vinglimidazol-Verbindungen erhältichen Verbindungen,
c) 0,05 bis 5 Gew.-% Ethylenglykoldi(meth)acrylat und/oder Pentaerythrittri-allylether,
d) 0 bis 95 Gew.-% Vinylpyrrolidon und/oder Vinylcaprolactam,
e) 0 bis 40 Gew.-% wenigstens einer Verbindung, die ausgewählt ist unter C₈-C₂₂-(Meth)acrylaten, C₈-C₂₂-Alkylvinylethern, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestern und Mischungen davon,
g) 0 bis 40 Gew.-% wenigstens eines Monomers, das ausgewählt ist unter Methyl(meth)acrylat, Ethyl(meth)acrylat, n-Butyl(meth)acrylat und Mischungen davon,
einpolymerisiert enthält,
wobei zur Copolymerisation Monomere mit ionogenen oder ionischen Gruppen in solchen Mengen eingesetzt werden, dass das Copolymer A) einen molaren Überschuss an anionogenen/anionischen Gruppen gegenüber kationogenen/kationischen Gruppen oder einen molaren Überschuss an kationogenen/kationischen Gruppen gegenüber anionogenen/anionischen Gruppen von wenigstens 2,5 : 1, aufweist.

Ein weiterer Gegenstand der Erfindung ist ein Verfahren zur Herstellung eines ampholytischen Copolymers A), wie im Folgenden definiert, durch radikalische Copolymerisation nach der Methode der Fällungspolymerisation.

Ein weiterer Gegenstand der Erfindung ist ein kosmetisches oder pharmazeutisches Mittel, enthaltend
A) wenigstens ein ampholytisches Copolymer wie zuvor und im Folgenden definiert,
B) wenigstens einen kosmetisch oder pharmazeutisch akzeptablen Wirk- oder Effektstoff, und
C) gegebenenfalls wenigstens einen weiteren, von B) verschiedenen kosmetisch oder pharmazeutisch akzeptablen Wirk- oder Hilfsstoff.

Insbesondere wird wenigstens ein Teil der Verbindungen a) und b) in Form eines Monomerenpaares eingesetzt wird, wobei das molare Verhältnis von anionogenen Gruppen der Komponente a) zu kationogenen Gruppen der Komponente b) 1:1 beträgt.

Die Herstellung der erfindungsgemäßen ampholytischen Copolymere A) erfolgt durch radikalische Copolymerisation nach der Methode der Fällungspolymerisation. In einer speziellen Ausführung werden zur Herstellung der erfindungsgemäßen Copolymere wenigstens zwei Radikalinitiatoren eingesetzt, deren Zerfallstemperaturen und/oder deren Halbwertszeiten bei einer bestimmten Polymerisationstemperatur voneinander verschieden sind. Dabei können Copolymere mit besonders geringen Restmonomergehalten erzielt werden. Dies ist insbesondere der Fall, wenn der bei höherer Temperatur zerfallende Initiator vor Abschluss, vorzugsweise vor Beginn der Fällung des Polymers, zugegeben wird.

Bei der Fällungspolymerisation sind die eingesetzten Monomere im Reaktionsmedium (Monomer, Lösungsmittel) löslich, das entsprechende Polymer aber nicht. Das entstehende Polymer wird unter den gewählten Polymerisationsbedingungen unlöslich und fällt aus dem Reaktionsgemisch aus.

Im Rahmen der vorliegenden Erfindung umfasst der Ausdruck Alkyl geradkettige und verzweigte Alkylgruppen. Geeignete kurzkettige Alkylgruppen sind z. B. geradkettige oder verzweigte C₁-C₇-Alkyl-, bevorzugt C₁-C₆-Alkyl-und besonders bevorzugt C₁-C₄-Alkylgruppen. Dazu zählen insbesondere Methyl, Ethyl, Propyl, Isopropyl, n-Butyl, 2-Butyl, sec.-Butyl, tert.-Butyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, Octyl etc.

Geeignete längerkettige C₈-C₃₀-Alkyl- bzw. C₈-C₃₀-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z. B. n-Hexyl(en), n-Heptyl(en), n-Octyl(en), n-Nonyl(en), n-Decyl(en), n-Undecyl(en), n-Dodecyl(en), n-Tridecyl(en), n-Tetradecyl(en), n-Pentadecyl(en), n-Hexadecyl(en), n-Heptadecyl(en), n-Octadecyl(en), n-Nonadecyl(en), Arachinyl(en), Behenyl(en), Lignocerinyl(en), Melissinyl(en), etc.

Cycloalkyl steht vorzugsweise für C₅-C₈-Cycloalkyl, wie Cyclopentyl, Cyclohexyl, Cycloheptyl oder Cyclooctyl.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Im Folgenden werden Verbindungen, die sich von Acrylsäure und Methacrylsäure ableiten können teilweise verkürzt durch Einfügen der Silbe "(meth)" in die von der Acrylsäure abgeleitete Verbindung bezeichnet.

Die erfindungsgemäßen Copolymere A) lassen sich unter Normalbedingungen (20 °C) vorteilhaft als Gele formulieren. "Gelförmige Konsistenz" zeigen Formulierungen, die eine höhere Viskosität als eine Flüssigkeit aufweisen und die selbsttragend sind, d. h. die eine ihnen verliehene Form ohne formstabilisierende Umhüllung behalten. Im Gegensatz zu festen Formulierungen lassen sich gelförmige Formulierungen jedoch leicht unter Anwendung von Scherkräften deformieren. Die Viskosität der gelförmigen Mittel liegt vorzugsweise in einem Bereich von größer als 600 bis etwa 60000 mPas, besonders bevorzugt von 6000 bis 30000 mPas. Vorzugsweise handelt es sich bei den Gelen um Haargele.

Unter wasserlöslichen Monomeren und Polymeren werden im Rahmen der vorliegenden Erfindung Monomere und Polymere verstanden, die sich zu mindestens 1 g/l bei 20 °C in Wasser lösen. Unter wasserdispergierbaren Monomeren und Polymeren werden Monomere und Polymere verstanden, die unter Anwendung von Scherkräften, beispielsweise durch Rühren, in dispergierbare Partikel zerfallen. Hydrophile Monomere sind vorzugsweise wasserlöslich oder zumindest wasserdispergierbar. Die erfindungsgemäßen Copolymere A) sind im Allgemeinen wasserlöslich.

Die erfindungsgemäßen ampholytischen Copolymere A) weisen sowohl anionogene und/oder anionische Gruppen, als auch kationogene und/oder kationische Gruppen auf. Zu ihrer Herstellung wird vorzugsweise ein Teil der Monomere a) und b) gemeinsam, d. h. in Form eines Monomerenpaares ("Monomerensalzes") eingesetzt. In dieser Monomerzusammensetzung beträgt das molare Verhältnis von anionogenen und anionischen Gruppen der Komponente a) zu kationogenen und kationischen Gruppen der Komponente b) 1:1 (d. h. einwertige Monomere werden im Wesentlichen äquimolar eingesetzt). Die Monomerenpaare können dabei vor ihrem Einsatz zur Polymerisation separat hergestellt werden. Bevorzugt ist jedoch die "in situ"-Herstellung der Monomerenpaare durch gemeinsamen Einsatz (z. B. gemeinsamen Zulauf) bei der Herstellung der Copolymerisate.

Vorzugsweise beträgt der Anteil des Monomerpaares an den zur Polymerisation eingesetzten Verbindungen wenigstens 1 Gew.-%, bevorzugt wenigstens 2 Gew.-%, insbesondere wenigstens 3 Gew.-%.

Weitere Monomere mit ionogenen/ionischen Gruppen, die nicht in Form des Monomerenpaares zur Polymerisation eingesetzt werden, werden vorzugsweise in zunächst nichtgeladenener Form, das heißt mit anionogenen oder kationogenen Gruppen eingesetzt. Gewünschtenfalls können zur Herstellung der erfindungsgemäßen ampholytischen Copolymere anstelle von ungeladenen Monomeren oder zusätzlich zu ungeladenen Monomeren oder zusätzlich zu den Monomerenpaaren auch bereits geladene Monomere, das heißt Monomere mit anionischen und kationischen Gruppen eingesetzt werden. Die Gegenionen, die diese Monomere tragen, leiten sich dann vorzugsweise von Säuren oder Basen ab, wie sie im Folgenden zur Einstellung des pH-Werts bei der Polymerisation oder der erhaltenen Polymerisate beschrieben werden. Kationische Monomere können zudem in teilweise oder vollständig quatemisierter Form eingesetzt werden.

Die Copolymere A) veisen einen deutlichen Überschuss an anionogenen/anionischen Gruppen oder an kationogenen/kationischen Gruppen auf. Erfindungs gemäβ werden zur Copolymerisation Monomere mit ionogenen oder ionischen Gruppen in solchen Mengen eingesetzt werden, dass das Copolymer A) einen molaren Überschuss an anionogenen/anionischen Gruppen gegenüber kationogenen/kationischen Gruppen oder einen molaren Überschuss an kationogenen/kationischen Gruppen gegenüber anionogenen/anionischen Gruppen von wenigstens 2,5 : 1, aufweist.

Die erfindungsgemäßen Copolymere A) enthalten insbesondere keine Siliciumatomhaltigen Gruppen eingebaut.

### Monomer a)

Die erfindungsgemäßen Copolymere A) enthalten als Verbindung a) Verbindung mit einer radikalisch polymerisierbaren α,β-ethylenisch ungesättigten Doppelbindung und mit mindestens einer anionogenen und/oder anionischen Gruppe pro 2 bis 96 Gew.-%, wie 2 bis 90 Gew.-%. bezogen auf das Gesamtgewicht der zur Polymerisation eingesetzten Verbindungen, Acrylsäure und/oder Methacrylsäure einpolymerisert. Die Komponente a) wird vorzugsweise in einer Menge von 2 bis 70 Gew.%, vorzugsweise 3 bis 60 Gew.-%, bezogen auf das Gesamtgewicht der zur Polymerisation eingesetzten Verbindungen (d. h. Komponenten a), b), c) und, falls vorhanden, d), e und g), eingesetzt.

### Monomer b)

Die erfindungsgemäßen Copolymere enthalten 2 bis 96 Gew.-%, besonders bevorzugt 2 bis 90 Gew.-%, insbesondere bevorzugt 3 bis 70 Gew.-%, bezogen auf das Gesamtgewicht der zur Polymerisation eingesetzten Monomere, wenigstens eines Monomers b) einpolymerisiert.

Erfindungs gemäβ enthält das Copolymer als Monomer b) wenigstens eine N-Vinylimidazol-Verbindung der allgemeinen Formel (II) 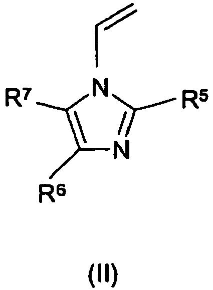 einpolymerisiert, worin R⁵ bis R⁷ unabhängig vonelnander für Wasserstoff, C₁-C₄-Alkyl oder Phenyl stehen.

Beispiele für Verbindungen der allgemeinen Formel (II) sind folgender Tabelle 1 zu entnehmen:

**Tabelle 1**

| **R⁵** | **R⁶** | **R⁷** |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl Ph = Phenyl | | |

Bevorzugt als Monomer b) ist 1-Vinylimidazol (N-Vinylimidazol).

Geeignete Monomere b) sind auch die durch Protonierung oder Quatemisierung der zuvor genannten N-Vinylimidazolverbindungen erhältlichen Verbindungen. Beispiele für solche geladenen Monomere b) sind quatemisierte Vinylimidazole, insbesondere 3-Methyl-1-vinylimidazoliumchlorid und -methosulfat. Geelgnete Säuren und Alkylierungsmittel sind carbonsäuren, wie Milchsäure, oder Mineralsäuren, wie Phosphorsäure, Schwefelsäure und Salzsäure, oder als Alkylierungsmittel C₁-C₄-Alkylhalogenide oder -sulfate, wie Ethylchlorid, Ethylbromid, Methylchlorid, Methylbromid, Dimethylsulfat und Diethylsulfat.

Eine spezielle Ausführung sind Copolymere A), die wenigstens eine N-Vinylimidazol-Verbindung, speziell N-Vinylimidazol, als alleiniges Monomer b) einpolymerisiert enthalten. Wird wenigstens eine N-Vinylimidazol-Verbindung, speziell N-Vinylimidazol, als alleiniges Monomer b) eingesetzt, so beträgt der Anteil vorzugsweise 3 bis 96 Gew.-%, bezogen auf das Gesamtgewicht der zur Polymerisation eingesetzten Verbindungen.

### Vernetzer c)

Die Copolymere A) enthalten wenigstens einen Vernetzer, d. h. eine Verbindung mit zwei oder mehr als zwei ethylenisch ungesättigten, nichtkonjugierten Doppelbindungen einpolymerisiert.

Vorzugsweise werden Vernetzer in einer Menge von 0,1 bis 4 Gew.-%, bezogen auf das Gesamtgewicht der zur Polymerisation eingesetzten Monomere, verwendet.

Weitere geeignete Vemetzer c) sind Divinyldioxan, Tetraallylsilan oder Tetravinylsilan.

Selbstverständlich können auch Mischungen der vorgenannten Verbindungen c) eingesetzt werden.

Erfindungs gemäβe Vernetzer c) sind Ethylenglykoldi(meth)acrylat und/oder Pentaerythrittriallylether.

### Monomer d)

In einer bevorzugten Ausführungsform enthalten die erfindungsgemäßen Copolymere zusätzlich zu den zuvor genannten Monomeren a) bis c)

N-Vinylpyrrolidon und/oder N-Vinylcaprolactam einpolymerisiert.

Die erfindungsgemäßen Copolymere A) enthalten vorzugsweise 5 bis 95 Gew.-%, besonders bevorzugt 10 bis 90 Gew.-%, bezogen auf das Gesamtgewicht der zur Polymerisation eingesetzten Verbindungen, wenigstens eines Monomers d) einpolymerisiert.

### Monomer e)

Die erfindungsgemäßen Copolymere A) enthalten vorzugsweise 0,2 bis 40 Gew.-%, besonders bevorzugt 0,5 bis 40 Gew.-%, insbesondere 1 bis 30 Gew.-%, bezogen auf das Gesamtgewicht der zur Polymerisation eingesetzten Verbindungen, wenigstens eines hydrophoben Monomers e) (und/oder wenigstens eines hydrophoben Monomers g) einpolymerisiert.

Geeignete Monomere e) sind z. B. n-Octyl(meth)acrylat,1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat und Mischungen davon.

Geeignete Monomere e) sind z. B. n-Octylvinylether, 1,1,3,3-Tetramethylbutylvinylether, Ethylhexylvinylether, n-Nonylvinylether, n-Decylvinylether, n-Undecylvinylether, Tridecylvinylether, Myristylvinylether, Pentadecylvinylether, Palmitylvinylether, Heptadecylvinylether, Octadecylvinylether, Nonadecylvinylether, Arrachinylvinylether, Behenylvinylether, Lignocerenylvinylether, Cerotinylvinylether, Melissinylvinylether, Palmitoleinylvinylether, Oleylvinylether, Linolylvinylether, Linolenylvinylether, Stearylvinylether, Laurylvinylether und Mischungen davon.

Geeignete Polyetheracrylate e) sind z. B. die Polykondensationsprodukte der zuvor genannten α,β-ethylenisch ungesättigten Mono- und/oder Dicarbonsäuren und deren Säurechloriden, -amiden und Anhydriden mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol R⁹-OH hergestellt werden. Die Alkylenoxide können einzeln, alternierend nacheinander oder als Mischung eingesetzt werden. Die Polyetheracrylate e) können allein oder in Mischungen zur Herstellung der erfindungsgemäß eingesetzten Polymere verwendet werden.

Geeignete Allylalkoholalkoxilate e) sind z. B. die Veretherungsprodukte von Allylchlorid mit entsprechenden Polyetherolen. Geeignete Polyetherole können leicht durch Umsetzung von Ethylenoxid, 1,2-Propylenoxid und/oder Epichlorhydrin mit einem Starteralkohol R⁹-OH hergestellt werden. Die Alkylenoxide können einzeln, altemierend nacheinander oder als Mischung eingesetzt werden. Die Allylalkoholalkoxilate e) können allein oder in Mischungen zur Herstellung der erfindungsgemäß eingesetzten Polymere verwendet werden.

Geeignete Monomere e) sind C₈-C₂₂-Carbonsäurevinylester. Dazu zählen z. B. n-Octylvinylester, 1,1,3,3-Tetramethylbutylvinylester, Ethylhexylvinylester, n-Nonylvinylester, n-Decylvinylester, n-Undecylvinylester, Tridecylvinylester, Myristylvinylester, Pentadecylvinylester, Palmitylvinylester, Heptadecylvinylester, Octadecylvinylester, Nonadecylvinylester, Arrachinylvinylester, Behenylvinylester, Lignocerenylvinylester, Cerotinylvinylester, Melissinylvinylester, Palmitoleinylvinylester. Oleylvinylester, Linolylvinylester, Linolenylvinylester, Stearylvinylester, Laurylvinylester und Mischungen davon.

>

### Monomer g)

Die erfindungsgemäßen Copolymere A) können zusätzlich wenigstens ein von den Komponenten a) bis e) verschiedenes, damit copolymerisierbares Monomer g) einpolymerisiert enthalten.

Erfindungs gemäß beträgt der Anteil an Monomeren g) bis zu 40 Gew.-%, bezogen auf das Gesamtgewicht der zur Polymerisation eingesetzten Verbindungen. Eine geeignete Einsatzmenge für zusätzliche Monomere g) liegt In einem Bereich von 0,1 bis 25 Gew.-%, insbesondere 0,5 bis 20 Gew.-%, bezogen auf das Gesamtgewicht der zur Polymerisation eingesetzten Verbindungen.

Besonders bevorzugte anionische ampholytischen Copolymere A) sind erhältlich durch radikalische Copolymerisation von
- wenigstens 2 Gew.-%, bezogen auf das Gesamtgewicht der zur Polymerisation eingesetzten Monomere, wenigstens eines Monomerenpaars aus N-Vinylimidazol und Acrylsäure und/oder Methacrylsäure,
- 5 bis 70 Gew.-% Methacrylsäure und/oder Acrylsäure,
- 0,1 bis 2 Gew.-% Ethylenglykol-di(meth)acrylat und/oder Pentaerythrittriallylether,
- 20 bis 95 Gew.-% Vinylpyrrolidon,
- 0 bis 40 Gew.-% wenigstens eines weiteren Monomers, das ausgewählt ist unter Methyl(meth)acrylat, Ethyl(meth)acrylat, n-Butyl(meth)acrylat, C₈-C₂₂-(Meth)acrylaten, C₈-C₂₂-Alkylvinylethern, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestern und Mischungen davon.

Anstelle von oder zusätzlich zu Vinylpyrrolidon kann auch Vinylcaprolactam eingesetzt werden.

Besonders bevorzugte anionische ampholytischen Copolymere A) sind weiterhin erhältlich durch radikalische Copolymerisation von
- wenigstens 5 Gew.-%, bezogen auf das Gesamtgewicht der zur Polymerisation eingesetzten Monomere, wenigstens eines Monomerenpaars aus N-Vinylimidazol und Acrylsäure und/oder Methacrylsäure,
- 5 bis 70 Gew.-% Methacrylsäure und/oder Acrylsäure,
- 0,1 bis 2 Gew.-% Ethylenglykol-di(meth)acrylat und/oder Pentaerythrittriallylether,
- 20 bis 85 Gew.-% Vinylpyrrolidon,
- 5 bis 40 Gew.-% wenigstens eines weiteren Monomers, das ausgewählt ist unter C₁-C₆-(Meth)acrylaten, insbesondere Methyl(meth)acrylat, Ethyl(meth)acrylat, n-Butyl(meth)acrylat und Mischungen davon.

Anstelle von oder zusätzlich zu Vinylpyrrolidon kann auch Vinylcaprolactam eingesetzt werden.

Besonders bevorzugte anionische ampholytischen Copolymere A) sind weiterhin erhältlich durch radikalische Copolymerisation von
- wenigstens 5 Gew.-%, bezogen auf das Gesamtgewicht der zur Polymerisation eingesetzten Monomere, wenigstens eines Monomerenpaars aus N-Vinylimidazol und Acrylsäure und/oder Methacrylsäure,
- 5 bis 70 Gew.-% Methacrylsäure und/oder Acrylsäure,
- 0,1 bis 2 Gew.-% Ethylenglykol-di(meth)acrylat und/oder Pentaerythrittriallylether,
- 20 bis 85 Gew.-% Vinylpyrrolidon,
- 1 bis 20 Gew.-% wenigstens eines weiteren Monomers, das ausgewählt ist unter C₈-C₃₀-(Meth)acrylaten, mit C₈-C₃₀-Alkylgruppen terminierten Polyether(meth)acrylaten und Mischungen davon, insbesondere unter Stearylmethacrylat, mit C₁₈-C₂₂-Alkylgruppen terminierten Polyethylenglycol(meth)-acrylaten und Mischungen davon.

Anstelle von oder zusätzlich zu Vinylpyrrolidon kann auch Vinylcaprolactam eingesetzt werden.

In einer speziellen Ausführung werden alle zuvor genannten anionischen ampholytischen Copolymere A), die wenigstens 5 Gew.-% mindestens einer Vinylimidazol-Verbindung einpolymerisiert enthalten, einer teilweisen oder vollständigen Quaternisierung unterzogen. Geeignete Quaternisierungsmittel sind die im Folgenden genannten.

Eine w eitere spezielle Ausführungsform der erfindungsgemäßen Copolymere A) sind kationisch ampholytische Copolymere.

Bevorzugte kationisch ampholytische Copolymere A) sind erhältlich durch radikalische Copolymerisation von
- Methacrylsäure und/oder Acrylsäure a),
- N-Vinylimidazol b),
- c) Ethylenglykoldi(meth)acrylat und/oder Pentaerythrittriallylether,
wobei das molare Verhältnis von kationogenen/kationischen Gruppen zu anionogenen/anionischen Gruppen wenigstens 6 : 1 beträgt.

Bevorzugte kationisch ampholytische Copolymere A) sind erhältlich durch radikalische Copolymerisation von
- Methacrylsäure und/oder Acrylsäure a),
- N-Vinylimidazol b),
- c) Ethylenglykoldi(meth)acrylat und/oder Pentaerythrittriallylether,
- 3 bis 35 Gew.-%, bezogen auf das Gesamtgewicht der zur Polymerisation eingesetzten Monomere, wenigstens eines Monomers g).
wobei das molare Verhältnis von kationogenen/kationischen Gruppen zu anionogenen/anionischen Gruppen wenigstens 6 : 1 beträgt.

Bevorzugte kationisch ampholytische Copolymere A) sind erhältlich durch radikalische Copolymerisation von
- Methacrylsäure und/oder Acrylsäure a),
- N-Vinylimidazol b),
- wenigstens einem Vernetzer c), vorzugsweise Ethylenglykoldi(meth)acrylat und/oder Pentaerythrittriallylether,
- 0,1 bis 10 Gew.-% wenigstens einer Verbindung e), die vorzugsweise ausgewählt ist unter C₈-C₂₂-(Meth)acrylaten, C₈-C₂₂-Alkylvinylethern, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestern und Mischungen davon,
wobei das molare Verhältnis von kationogenen/kationischen Gruppen zu anionogenen/anionischen Gruppen wenigstens 6 : 1 beträgt.

Besonders bevorzugte kationische ampholytischen Copolymere A) sind erhältlich durch radikalische Copolymerisation von
- wenigstens 2 Gew.-%, bezogen auf das Gesamtgewicht der zur Polymerisation eingesetzten Monomere, wenigstens eines Monomerenpaars aus N-Vinylimidazol und Acrylsäure und/oder Methacrylsäure,
- 3 bis 70 Gew.-% wenigstens einen
- 0,1 bis 2 Gew.-% Ethylenglykol-di(meth)acrylat und/oder Pentaerythrittriallylether,
- 0 bis 95 Gew.-%, vorzugsweise 20 bis 95 Gew.-% Vinylpyrrolidon und/oder Vinylcaprolactam,
- 0 bis 40 Gew.-% wenigstens eines weiteren Monomers, das ausgewählt ist unter Methyl(meth)acrylat, Ethyl(meth)acrylat, n-Butyl(meth)acrylat, C₈-C₂₂-(Meth)acrylaten, C₈-C₂₂-Alkylvinylethern, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestern und Mischungen davon.

Besonders bevorzugte kationische ampholytischen Copolymere A) sind weiterhin erhältlich durch radikalische Copolymerisation von
- wenigstens 2 Gew.-%, bezogen auf das Gesamtgewicht der zur Polymerisation eingesetzten Monomere, wenigstens eines Monomerenpaars aus N-Vinylimidazol und Acrylsäure und/oder Methacrylsäure, r
- 3 bis 50 Gew.-% wenigstens einen Vinylimidazol-Verbindung,
- 0,1 bis 2 Gew.-% Ethylenglykol-di(meth)acrylat und/oder Pentaerythrittriallylether,
- 20 bis 95 Gew.-% Vinylpyrrolidon und/oder Vinylcaprolactam;
- 5 bis 40 Gew.-% wenigstens eines weiteren Monomers, das ausgewählt ist unter Methyl(meth)acrylat, Ethyl(meth)acrylat. n-Butyl(meth)acrylat und Mischungen davon.

Besonders bevorzugte kationische ampholytischen Copolymere A) sind weiterhin erhältlich durch radikalische Copolymerisation von
- wenigstens 5 Gew.-%, bezogen auf das Gesamtgewicht der zur Polymerisation eingesetzten Monomere, wenigstens eines Monomerenpaars aus N-Vinylimidazol und Acrylsäure und/oder Methacrylsäure,
- 3 bis 70 Gew.-% wenigstens einen Vinylimidazol-Verbindung,
- 0,1 bis 2 Gew.-% Ethylenglykol-di(meth)acrylat und/oder Pentaerythrittriallylether,
- 20 bis 85 Gew.-% Vinylpyrrolidon und/oder Vinylcaprolactam,
- 1 bis 20 Gew.-% wenigstens eines weiteren Monomers, das ausgewählt ist unter C₈-C₃₀-(Meth)acrylaten, mit C₈-C₃₀-Alkylgruppen terminierten Polyether(meth)acrylaten und Mischungen davon, insbesondere unter Stearylmethacrylat, mit C₁₈-C₂₂-Alkylgruppen terminierten Polyethylenglycol(meth)-acrylaten und Mischungen davon.

In einer speziellen Ausführung werden alle zuvor genannten kationischen ampholytischen Copolymere A) einer teilweisen oder vollständigen Quatemisierung unterzogen. Geeignete Quaternisierungsmittel sind die im Folgenden genannten.

In einer speziellen Ausführung erfolgt die radikalische Copolymerisation der zuvor genannten Komponenten a) bis c) und, falls vorhanden, d) bis g) in Gegenwart wenigstens einer polyetherhaltigen Verbindung, die keine copolymerisierbare Doppelbindung aufweist. Dabei werden spezielle ampholytische Copolymere mit vorteilhaften Eigenschaften erhalten. Dies kann beispielsweise auf eine Wirkung der Polyetherkomponente als Schutzkolloid oder Emulgator zurückzuführen sein. Dies kann beispielsweise auch aus einer zumindest teilweisen Pfropfung auf die Polyetherkomponente als Pfropfgrundlage resultieren. Es sind jedoch auch andere Mechanismen als eine Pfropfung vorstellbar. Die erfindungsgemäßen ampholytischen Copolymere umfassen ganz allgemein die Verfahrensprodukte der radikalischen Copolymerisation, worunter z. B. reine Pfropfpolymerisate, Mischungen von Pfropfpolymerisaten mit ungepfropften Verbindungen der Polyetherkomponente sowie beliebige Mischungen verstanden werden.

Vorzugsweise beträgt die Einsatzmenge der Polyetherkomponente (falls vorhanden) 0,1 bis 50 Gew.-%, besonders bevorzugt 1 bis 25 Gew.-%, bezogen auf das Gesamtgewicht der zur Polymerisation eingesetzten Komponenten.

Geeignete polyetherhaltige Verbindungen sind beispielsweise wasserlösliche oder wasserdispergierbare nichtionische Polymere, die Alkylenoxid-Wiederholungseinheiten aufweisen. Vorzugsweise beträgt der Anteil an Alkylenoxid-Wiederholungseinheiten mindestens 30 Gew.-%, bezogen auf das Gesamtgewicht der Verbindung. Geeignete polyetherhaltige Verbindungen sind beispielsweise Polyalkylenglykole, wie sie üblicherweise auch als nichtionische Tenside eingesetzt werden. Geeignete Polyalkylenglykole weisen im Allgemeinen ein zahlenmittleres Molekulargewicht im Bereich von etwa 150 bis 100000, bevorzugt 300 bis 50000, besonders bevorzugt 500 bis 40000 auf. Geeignete Polyalkylenglykole sind beispielsweise Polyethylenglykole, Polypropylenglykole, Polytetrahydrofurane und Alkylenoxidcopolymere. Geeignete Alkylenoxide zur Herstellung von Alkylenoxidcopolymeren sind z. B. Ethylenoxid, Propylenoxid, Epichlorhydrin, 1,2- und 2,3-Butylenoxid. Die Alkylenoxidcopolymere können die Alkylenoxideinheiten statistisch verteilt oder in Form Blöcken einpolymerisiert enthalten. Vorteilhafterweise verwendet man Homopolymerisate des Ethylenoxids oder Copolymerisate, die Ethylenoxid enthalten. Vorzugsweise beträgt der Anteil an von Ethylenoxid abgeleiteten Wiederholungseinheiten 40 bis 99 Gew.-%. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid.

Die Herstellung der Copolymere A1) durch Fällungspolymerisation.

Die Fällungspolymerisation erfolgt vorzugsweise in einem wasserfreien, aprotischen Lösungsmittel oder Lösungsmittelgemisch, vorzugsweise in Ethylacetat und/oder n-Butylacetat. Bevorzugt erfolgt die Fällungspolymerisation bei einer Temperatur im Bereich von 70 bis 140°C, bevorzugt 75 bis 100°C, insbesondere von 80 bis 95 °C. Die resultierenden Polymerteilchen fallen aus der Reaktionslösung aus und können durch übliche Verfahren, wie Filtration mittels Unterdruck, isoliert werden. Zur Fätlungspolymerisation können oberflächenaktive, polymere Verbindungen, vorzugsweise auf Polysiloxanbasis, eingesetzt werden. Bei der Fällungspolymerisation werden in der Regel Polymere mit höheren Molekulargewichten als bei der Lösungspolymerisation erhalten.

Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 5 bar.

Zur Herstellung der Polymerisate können die Monomeren mit Hilfe von Radikale bildenden Initiatoren polymerisiert werden.

Als Initiatoren für die radikalische Polymerisation können die hierfür üblichen Peroxo- und/oder Azo-Verbindungen eingesetzt werden, beispielsweise Alkalh oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-amylperoxid, tert.-Butylhydroperoxid, 2,2'-Azo-bis-isobutyronitril, Azo-bis-(2-amidinopropan)dihydrochlorid oder 2,2'-Azo-bis-(2-methyl-butyronitril). Geeignet sind auch Initiatormischungen oder Redox-Initiator-Systeme, wie z. B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat, H₂O₂/Cu¹.

In einer speziellen Ausführung werden zur Herstellung der erfindungsgemäßen Copolymere wenigstens zwei Radikalinitiatoren eingesetzt, die eine im Wesentlichen unabhängige Initiierung in wenigstens zwei Phasen ermöglichen. Dabei können Copolymere mit besonders geringen Restmonomergehalten erzielt werden.

Bevorzugt werden zur Copolymerisation wenigstens zwei initiatoren eingesetzt, deren Zerfallstemperaturen um wenigstens 10°C voneinander verschieden sind. Im Rahmen der Erfindung ist die Zerfallstemperatur ist definiert als die Temperatur, bei der 50 % der Moleküle innerhalb von 2,5 Stunden in freie Radikale zerfallen. Vorzugsweise erfolgt die Copolymerisation bei dieser Vorgehensweise bis zum Abschluss der Fällung des Copolymers bei einer Temperatur größter oder gleich der niedrigeren Zerfallstemperatur und kleiner der höheren Zerfallstemperatur, und nach der Fällung erfolgt eine weitere Umsetzung bei einer Temperatur größer oder gleich der höheren Zerfallstemperatur.

Bevorzugt umfasst das erfindungsgemäße Verfahren eine erste Polymerisationsphase bei einer ersten Polymerisationstemperatur und eine zweite Polymerisationsphase bei einer zweiten Polymerisationstemperatur oberhalb der ersten Polymerisationstemperatur, wobei zur Polymerisation wenigstens zwei Initiatoren eingesetzt werden, deren Halbwertszeiten bei der ersten Polymerisationstemperatur sich so unterscheiden, dass wenigstens einer dieser Initiatoren während der ersten Polymerisationsphase in Radikale zerfällt und wenigstens einer dieser Initiatoren während der ersten Polymerisationsphase im Wesentlichen nicht in Radikale zerfällt und während der zweiten Polymerisationsphase in Radikale zerfällt. Vorzugsweise beginnt bei dieser Vorgehensweise die zweite Polymerisationsphase im Wesentlichen nach Fällung des Copolymers. Unter "im Wesentlichen" nach Fällung des Copolymers wird verstanden, dass das Copolymer vorzugsweise zu mindestens 80 Gew.-%, vorzugsweise mindestens 90 Gew.-%, insbesondere wenigstens 95 Gew.-%, bezogen auf das Gesamtgewicht des Copolymers, in gefällter Form vorliegt.

Die Halbwertszeit eines Initiators kann nach üblichen, dem Fachmann bekannten Ver fahren bestimmt werden, wie z. B. in der Druckschrift "Initiators for high polymers", Akzo Nobel, Nr. 10737, beschrieben. Vorzugsweise liegt die Halbwertszeit des ersten Polymerisationsinitiators bei der ersten Polymerisationstemperatur und des zweiten Polymerisationsinitiators bei der zweiten Polymerisationstemperatur in einem Bereich von etwa 1 Minute bis 3 Stunden, besonders bevorzugt 5 Minuten bis 2,5 Stunden. Gewünschtenfalls können auch kürzere Halbwertszeiten z. B. von 1 Sekunde bis 1 Minute oder längere Halbwertszeiten als 3 Stunden zum Einsatz kommen, solange sichergestellt ist, dass der/die bei der höheren Temperatur zerfallende(n) Initiator(en) im Wesentlichen während der zweiten Polymerisationsphase in Radikale zerfällt.

Zusätzlich zu der ersten und zweiten Polymerisationsphase können weitere Polymerisationsphasen bei davon verschiedenen Polymerisationstemperaturen angewandt werden. So ist es z. B. möglich, eine erste Polymerisationsphase bei einer ersten Polymerisationstemperatur durchzuführen, die so gewählt ist, dass eine kontrollierte Polymerisation (d. h. z. B. unter Vermeidung eines unerwünschten Temperaturanstiegs durch die Reaktionswärme, einer zu hohen Reaktionsgeschwindigkeit, etc.) erfolgt. Anschließend kann sich z. B. eine Nachpolymerisation bei einer Temperatur anschließen, die oberhalb der ersten und unterhalb der zweiten Polymerisationstemperatur liegt und die so gewählt ist, dass der/die bei der höheren Temperatur zerfallende(n) Initiator(en) im Wesentlichen nicht in Radikale zerfallen. Nach Abschluss dieser Nachpolymerisation, zu der gewünschtenfalls nochmals der bei der niedrigeren Temperatur zerfallende Initiator und/oder ein anderer unter den Bedingungen der Nachpolymerisation zerfallender Initiator zugegeben werden kann, kann sich dann die zweite Polymerisationsphase anschließen.

Vorzugsweise enthält das eingesetzte Initiatorsystem wenigstens zwei Initiatoren, deren Zerfallstemperaturen sich um wenigstens 15 °C voneinander unterscheiden.

Der bei der niedrigeren Temperatur zerfallende Initiator weist vorzugsweise eine Zerfallstemperatur von 50 bis 100 °C auf.

Der bei der höheren Temperatur zerfallende Initiator weist vorzugsweise eine Zerfallstemperatur von 80 bis 150 °C auf.

Vorzugsweise wird der bei der höheren Temperatur zerfallende Initiator zu Beginn der Copolymerisation vorgelegt oder vor oder während der Fällung des Copolymers zugegeben.

Vorzugsweise wird der bei der höheren Temperatur zerfallende Initiator zu Beginn der Copolymerisation vorgelegt oder vor der Fällung des Copolymers zugegeben.

Bei einer bevorzugten Initiatorkombination handelt es sich bei dem bei der niedrigeren Temperatur zerfallenden Initiator um Trigonox® EHP (Bis(2-ethylhexyl)peroxydicarbonat, CAS-Nr. 16111-62-9) und ist der bei der höheren Temperatur zerfallende Initiator ausgewählt unter tert.-Butylperoxypivalat (z. B. Luperox 11 M75 der Fa. Atochem), tert.-Butylperoctoat, Lauroylperoxid (LPO, CAS-Nr. 105-74-8) oder 2,5-Dimethyl-2,5-bis(t-butylperoxy)hexan (Trigonox® 101).

Zur Erzielung möglichst reiner Polymere können die Polymere z. B. einem Waschschritt mit einem geeigneten Lösungsmittel, z. B. dem auch zur Polymerisation eingesetzten Lösungsmittel unterzogen werden.

Die anionogenen Gruppen (Säuregruppen) der Copolymere A) können mit einer Base teilweise oder vollständig neutralisiert werden. Als Base für die Neutralisation der Polymere können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxid, Calciumoxid, Magnesiumhydroxid oder Magnesiumcarbonat sowie Amine verwendet werden. Geeignete Amine sind z. B. C₁-C₆-Alkylamine, bevorzugt n-Propylamin und n-Butylamin, Dialkylamine, bevorzugt Diethylpropylamin und Dipropylmethylamin, Trialkylamine, bevorzugt Triethylamin und Triisopropylamin. Bevorzugt sind Aminoalkohole, z. B. Trialkanolamine, wie Triethanolamin, Alkyldialkanolamine, wie Methyl- oder Ethyldiethanolamin und Dialkylalkanolamine, wie Dimethylethanolamin sowie 2-Amino-2-methyl-1-propanol. Besonders für den Einsatz in Haarbehandlungsmitteln haben sich zur Neutralisation der Säuregruppen enthaltenden Polymere NaOH, KOH, 2-Amino-2-methyl-1-propanol, 2-Amino-2-ethylpropan-1,3-diol, Diethylaminopropylamin und Triisopropanolamin bewährt. Die Neutralisation der Säuregruppen kann auch mit Hilfe von Mischungen mehrerer Basen vorgenommen werden, z. B. Mischungen aus Natronlauge und Triisopropanolamin. Die Neutralisation kann je nach Anwendungszweck partiell oder vollständig erfolgen.

Geladene kationische Gruppen lassen sich aus den vorliegenden kationogenen stickstoffhaltigen Gruppen entweder durch Protonierung, z. B. mit ein- oder mehrwertigen Carbonsäuren, wie Milchsäure oder Weinsäure, oder mit Mineralsäuren, wie Phosphorsäure, Schwefelsäure und Salzsäure, oder durch Quatemisierung, z. B. mit Alkylierungsmitteln, wie C₁-C₄-Alkylhalogeniden oder -sulfaten, erzeugen. Beispiele solcher Alkylierungsmittel sind Ethylchlorid, Ethylbromid, Methylchlorid, Methylbromid, Dimethylsulfat und Diethylsulfat.

Sollen die erfindungsgemäßen Copolymere A) sowohl quatemisiert als auch neutralisiert werden, so erfolgt vorzugsweise zuerst die Quaternisierung und anschließend die Neutralisierung.

Die erfindungsgemäßen ampholytischen Copolymere A) eignen sich in vorteilhafter Weise zur Modifizierung der rheologischen Eigenschaften wässriger Wirk- oder Effektstoffzusammensetzungen. Dabei kann es sich ganz allgemein beispielsweise um kosmetische Zusammensetzungen, pharmazeutische Zusammensetzungen, Hygieneprodukte, Anstrichmittel, Zusammensetzungen für die Papierindustrie sowie die Textilindustrie handeln. In einer bevorzugten Ausführungsform enthalten die Zusammensetzungen wenigstens einen wasserlöslichen oder zumindest wasserdispergierbaren Wirk- oder Effektstoff. Selbstverständlich eignen sich die erfindungsgemäßen Copolymere A) auch zur Modifizierung der rheologischen Eigenschaften von Zusammensetzungen, die wenigstens eine wasserunlöslichen (hydrophoben) Wirk- oder Effektstoff enthalten.

"Modifizierung rheologischer Eigenschaften" wird im Rahmen der vorliegenden Erfindung weit verstanden. Die erfindungsgemäß verwendeten Copolymere A) eignen sich im Allgemeinen zur Verdickung der Konsistenz von wässrigen Zusammensetzungen in einem weiten Bereich. Je nach Grundkonsistenz der flüssigen Zusammensetzungen können in Abhängigkeit von der Einsatzmenge des Copolymers A) in der Regel Fließeigenschaften von dünnflüssig bis hin zu fest (im Sinne von "nicht mehr fließend") erzielt werden. Unter "Modifizierung rheologischer Eigenschaften" wird daher u. a. die Erhöhung der Viskosität von Flüssigkeiten, die Verbesserung der Tixotropie-Eigenschaften von Gelen, die Verfestigung von Gelen und Wachsen etc. verstanden. Die erfindungsgemäßen Mittel eignen sich vorzugsweise zur Formulierung von wässrigen kosmetischen und pharmazeutischen Produkten. Vorzugsweise sind dabei die Zusammensetzungen der Copolymere A) im Allgemeinen klar. Vorteilhafterweise können somit Formulierungen, insbesondere kosmetische Formulierungen, ohne Beeinträchtigung durch die Eigenfarbe der Zusammensetzungen angefärbt werden. Des Weiteren können die Zusammensetzungen in Form von opaken bis klaren Gelen formuliert werden.

Die erfindungsgemäßen ampholytischen Copolymere A) eignen sich speziell als Rheologiemodifizierungsmittel mit über den pH-Wert steuerbaren Eigenschaften. So eignen sich z. B. die zuvor genannten anionischen ampholytischen Copolymere A) als pH-schaltbare Verdicker für einen pH-Bereich größer oder gleich 6. Die zuvor genannten kationischen ampholytischen Copolymere A) eignen sich als pH-schaltbare Verdicker für einen pH-Bereich kleiner oder gleich 6,5. Quaternisierte ampholytischen Copolymere A), die im Wesentlichen keine protonierbaren Gruppen aufweisen, eignen sich unabhängig vom pH-Wert als Rheologiemodifizierungsmittel in einem pH-Bereich von etwa 2 bis 10.

Die erfindungsgemäßen ampholytischen Copolymere A) eignen sich speziell auch als Rheologiemodifizierungsmittel für salzhaltige Zusammensetzungen.

Vorteilhafterweise wirken die erfindungsgemäßen ampholytischen Copolymere A) auch als filmbildende und/oder konditionierend wirkende Rheologiemodifizierungsmittel. Sie eignen sich somit speziell für kosmetische und dermatologische Mittel, speziell in Haarfestigem als "festigende Verdicker" und in Haarpflegemitteln als "konditionierende Verdicker".

Die ampholytischen Copolymere A) eignen sich sowohl zur Herstellung homogenphasiger wässriger Zusammensetzungen als auch zur Formulierung von heterogenphasigen Zusammensetzungen, die zusätzlich wenigstens eine wasserunlösliche (hydrophobe) flüssige oder feste Verbindung umfassen. "Homogenphasige Zusammensetzungen" weisen unabhängig von der Anzahl ihrer Bestandteile nur eine einzige Phase auf. "Heterogenphasige Zusammensetzungen" sind disperse Systeme von zwei oder mehreren miteinander nicht mischbaren Komponenten. Dazu zählen fest/flüssig-, flüssig/flüssig- und fest/flüssig/flüssig-Zusammensetzungen, wie Dispersionen und Emulsionen, z. B. O/W- und W/O-Formulierungen, die wenigstens eine der im Folgenden näher beschriebenen Öl- bzw. Fettkomponenten und Wasser als nicht mischbare Phasen aufweisen. Prinzipiell können die Copolymere A) sowohl in der Wasserphase als auch in der Ölphase eingesetzt werden. Im Allgemeinen enthalten heterogenphasige flüssig/flüssig-Zusammensetzungen die Copolymere A) im Wesentlichen in der Wasserphase.

Die erfindungsgemäßen Copolymere A) eignen sich weiterhin als Solubilisator für im Wesentlichen wasserunlösliche Verbindungen. Ein weiterer Gegenstand der Erfindung ist daher die Verwendung eines ampholytischen Copolymers A), wie zuvor definiert, als Solubilisator zur Herstellung wässriger Formulierungen von Wirk- und Effektstoffen, die in Wasser bei 25 °C und 1013 mbar eine Löslichkeit unterhalb von 10 g/l aufweisen.

Des Weiteren wurde gefunden, dass sich die erfindungsgemäßen Copolymere A) in vorteilhafter Weise als Schutzkolloid eignen. Ein weiterer Gegenstand der Erfindung ist daher die Verwendung eines ampholytischen Copolymers A), wie zuvor definiert, als Schutzkolloid bei der radikalischen wässrigen Emulsionspolymerisation.

Auf Grund der zuvor genannten Eigenschaften eignen sich die erfindungsgemäßen Copolymere A) ganz allgemein zur Herstellung von Wirk- oder Effektstoffzusammensetzungen, enthaltend
A) wenigstens ein ampholytisches Copolymer, wie zuvor definiert,
B) wenigstens einen Wirk- oder Effektstoff und
C) gegebenenfalls wenigstens einen weiteren, von A) und B) verschiedenen Wirk-oder Hilfsstoff.

Wirkstoffe für Kosmetika, Arzneimittel, Hygienemittel, Textilbehandlungsmittel etc., d. h. Substanzen, die im Allgemeinen bereits in geringer Konzentration eine Wirkung entfalten, z. B. eine kosmetische Wirkung auf Haut und/oder Haaren, eine pharmakologische Wirkung in einem Organismus, eine reinigende und/oder desinfizierende Wirkung, eine Modifizierung eines textilen Stoffes, z. B. eine knitterfreie Ausrüstung, sowie Effektstoffe, die Lebewesen oder unbelebten Substraten eine bestimmte Eigenschaft verleihen, beispielsweise Farbpigmente für Schminken oder Dispersionsfarben, werden häufig in Form wässriger Wirk- oder Effektstoffzusammensetzungen formuliert und angewendet.

Die Wirk- und Effektstoffzusammensetzungen enthalten die Polymerkomponente A) vorzugsweise in einem Anteil von etwa 0,001 bis 50 Gew.-%, besonders bevorzugt 0,01 bis 30 Gew.-%, insbesondere 0,1 bis 20 Gew.-%, bezogen auf das Gesamtgewicht des Mittels.

Die Komponenten B) und C) werden nach dem gewünschten Einsatzbereich der Zusammensetzung ausgewählt. Neben einsatzbereichtypischen Komponenten (z. B. bestimmten pharmazeutischen Wirkstoffen) sind sie ausgewählt unter Trägem, Exzipienten, Emulgatoren, Tensiden, Konservierungsmitteln, Duftstoffen, von Komponente A) verschiedenen Verdickern, Polymeren, Gelbildnern, Farbstoffen, Pigmenten, Lichtschutzmitteln, Konsistenzgebem, Antioxidantien, Entschäumern, Antistatika, Harzen, Lösemitteln, Lösungsvermittlern, Neutralisierungsmitteln, Stabilisatoren, Sterilantien, Treibmitteln, Trocknungsmitteln, Trübungsmitteln, etc.

Vorzugsweise weisen die Zusammensetzungen eine Trägerkomponente C) auf, die ausgewählt ist unter Wasser, hydrophilen Komponenten, hydrophoben Komponenten und Mischungen davon.

Geeignete hydrophile Träger C) sind z. B. ein-, zwei- oder mehrwertige Alkohole mit vorzugsweise 1 bis 8 Kohlenstoffatomen, wie Ethanol, n-Propanol, Isopropanol, Propylenglycol, Glycerin, Sorbit, etc.

Geeignete hydrophobe Träger C) sind vorzugsweise ausgewählt unter
i) Ölen, Fetten, Wachsen,
ii) von iii) verschiedenen Estern von C₆-C₃₀-Monocarbonsäuren mit ein-, zwei- oder dreiwertigen Alkoholen,
iii) gesättigten acyclischen und cyclischen Kohlenwasserstoffen,
iv) Fettsäuren,
v) Fettalkoholen,
vi) Treibgasen,
und Mischungen davon.

Geeignete Siliconöle C) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten C) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkemöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete kosmetisch und pharmazeutisch verträgliche Öl- bzw. Fettkomponenten C) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Die erfindungsgemäßen Zusammensetzungen können als Wirkstoff, z. B. als kosmetischen und/oder pharmazeutischen Wirkstoff B) (wie auch gegebenenfalls als Hilfsstoff C)) wenigstens ein Polymer enthalten, das sich von den erfindungsgemäßen ampholytischen Copolymeren A) unterscheidet. Dazu zählen ganz allgemein anionische, kationische, amphotere und neutrale Polymere.

Beispiele für anionische Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane, z. B. Luviset PUR® der Fa. BASF, und Polyharnstoffe. Besonders geeignete Polymere sind Copolymere aus t-Butylacrylat, Ethylacrylat, Methacrylsäure (z. B. Luvimer® 100P), Copolymere aus Ethylacrylat und Methacrylsäure (z. B. Luvimer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vnylester (z. B. Luviset®-Marken), Maleinsäureanhydridcopolymere, gegebenenfalls mit Alkohol umgesetzt, anionische Polysiloxane, z. B. carboxyfunktionelle, t-Butylacrylat, Methacrylsäure (z. B. Luviskol® VBM), Copolymere von Acrylsäure und Methacrylsäure mit hydrophoben Monomeren, wie z. B. C₄-C₃₀-Alkylester der (Meth)acrylsäure, C₄-C₃₀-Alkylvinylester, C₄-C₃₀-Alkylvinylether und Hyaluronsäure. Beispiele für anionische Polymere sind weiterhin Vinylacetat/- Crotonsäure-Copolymere, wie sie beispielsweise unter den Bezeichnungen Resyn® (National Starch) und Gafset® (GAF) im Handel sind und Vinylpyrrolidon/Vinylacrylat-Copolymere, erhältlich beispielsweise unter dem Warenzeichen Luviflex® (BASF). Weitere geeignete Polymere sind das unter der Bezeichnung Luviflex® VBM-35 (BASF) erhältliche Vinylpyrrolidon/Acrylat-Terpolymer und Natriumsulfonat-haltige Polyamide oder Natriumsulfonat-haltige Polyester. Weiterhin geeignet sind Vinylpyrrolidon/Ethylmethacrylat/Methacrylsäure-Copolymere wie sie von der Fa. Stepan unter den Bezeichnungen Stepanhold-Extra und -R1 vertrieben werden und die Carboset®-Marken der Fa. BF Goodrich.

Geeignete kationische Polymere sind z. B. kationische Polymere mit der Bezeichnung Polyquaternium nach INCI, z. B. Copolymere aus Vinylpyrrolidon/N-Vinylimidazoliumsalzen (Luviquat® FC, Luviquat® HM, Luviquat® MS, Luviset Clear®, Luviquat Supreme®, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidocopolymere (Polyquaternium-7) und Chitosan. Geeignete kationische (quaternisierte) Polymere sind auch Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar®-Marken der Fa. Rhodia.

Ganz besonders geeignete Polymere sind neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex® Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol® Plus (BASF), oder Polyvinylpyrrolidon und deren Copolymere, insbesondere mit Vinylestern, wie Vinylacetat, z. B. Luviskol® VA 37 (BASF); Polyamide, z. B. auf Basis von Itaconsäure und aliphatischen Diaminen, wie sie z. B. in der DE-A-43 33 238 beschrieben sind.

Geeignete Polymere sind auch amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/- Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).

Geeignete Polymere sind auch nichtionische, siloxanhaltige, wasserlöslichen oder -dispergierbare Polymere, z. B. Polyethersiloxane, wie Tegopren® (Fa. Goldschmidt) oder Belsil® (Fa. Wacker).

Wie bereits ausgeführt, sind die Copolymere A) in vorteilhafter Weise geeignet, wasserunlösliche (bzw. nur gering wasserlösliche) Wirk- und Effektstoffe B) in wässriger Phase zu stabilisieren und ermöglichen daher die Herstellung wässriger Formulierungen derartiger Wirk- und Effektstoffe. Sie eignen sich auch zur Herstellung fester Formulierungen dieser Wirk- und Effektstoffe, die sich in eine wässrige Formulierung, z. B. als Handels-, Darreichung- oder Wirkform, überführen lassen. Dies kann auch erst nach Applikation der feste Zusammensetzung erfolgen (z. B. im Verdauungstrakt eines Organismus, etc.).

Die mit den erfindungsgemäß eingesetzten Polymeren A) erzielte "Löslichkeitsverbesserung" wird im Rahmen der vorliegenden Erfindung daher breit verstanden. Dazu zählt zum einen die Stabilisierung heterogener Systeme, bei denen der Wirkstoff als emulgierte bzw. dispergierte Phase (disperse Phase) in einem wässrigen Medium als kontinuierliche Phase vorliegt. Dazu zählt weiterhin die Stabilisierung von Übergangsstufen zu homogenen Lösungen, wie kolloidale Lösungen, etc., bis hin zu molekular dispersen Lösungen. Dazu zählt auch eine Löslichkeitsverbesserung im Sinne einer Solubilisierung, bei der die schlecht wasserlöslichen oder wasserunlöslichen Stoffe in klare, höchstens opaleszierende wässrige Lösungen überführt werden. Dazu zählt schließlich auch die Befähigung zur Bildung so genannter "fester Lösungen".

Eine geringe (schlechte) Löslichkeit bedeutet im Rahmen dieser Erfindung eine Löslichkeit des Wirk- bzw. Effektstoffs in Wasser von unterhalb 10 g/l, insbesondere unterhalb 1 g/l und speziell unterhalb 0,1 g/l bei 25°C und 1013 mbar.

Die unter Verwendung der Copolymere A) hergestellten wässrigen Wirkstoffzusammensetzungen von in Wasser unlöslichen Wirk- bzw. Effektstoffen umfassen neben einem wässrigen Medium als kontinuierliche Phase wenigstens einen in der kontinuierlichen Phase solubilisierten oder dispergierten Wirkstoff und/oder Effektstoff B), der in Wasser bei 25 °C/1013 mbar eine Löslichkeit unterhalb 10 g/l, insbesondere unterhalb 1 g/l und speziell unterhalb 0,1 g/l aufweisen, sowie wenigstens ein ampholytisches Copolymer A).

Der Wirkstoff liegt in der kontinuierlichen wässrigen Phase in äußerst feinteiliger Form vor. Dies kann beispielsweise darauf zurückzuführen sein, dass der Wirkstoff in der wässrigen Phase mit den Polymeren A) Aggregate bildet. Diese Aggregate weisen in der Regel mittlere Teilchengrößen unterhalb 1 µm, häufig unterhalb 500 nm, insbesondere unterhalb 400 nm, speziell unterhalb 300 nm auf. Je nach Art des Polymeren und des Wirkstoffs bzw. Effektstoffs sowie abhängig von den Konzentrationsverhältnissen können die Aggregate auch so klein werden, dass sie nicht mehr in Form nachweisbarer, diskreter Partikel sondern in gelöster Form vorliegen (Teilchengröße < 10 nm).

Die hier angegebenen Teilchengrößen sind gewichtsmittlere Teilchengrößen, wie sie durch dynamische Lichtstreuung ermittelt werden können. Verfahren hierzu sind dem Fachmann geläufig, beispielsweise aus H. Wiese in D. Distler, Wässrige Polymerdispersionen, Wiley-VCH 1999, Kapitel 4.2.1, S. 40ff und dort zitierte Literatur sowie H. Auweter, D. Horn, J: Colloid Interf. Sci. 105 (1985) 399, D. Lilge, D. Horn, Colloid Polym. Sci. 269 (1991) 704 oder H. Wiese, D. Horn, J. Chem. Phys. 94 (1991) 6429.

Vorteilhaft können die Copolymere A) als Solubilisatoren für in Wasser schwerlösliche oder unlösliche UV-Absorber verwendet werden.

Der Betriff UV-Absorber umfasst im Rahmen der vorliegenden Erfindung UV-A-, UV-Bund/oder Breitbandfilter.

Vorteilhafte Breitbandfilter, UV-A- oder UV-B-Filtersubstanzen sind beispielsweise Vertreter der folgenden Verbindungsklassen:

Bis-Resorcinyltriazinderivate mit der folgenden Struktur: 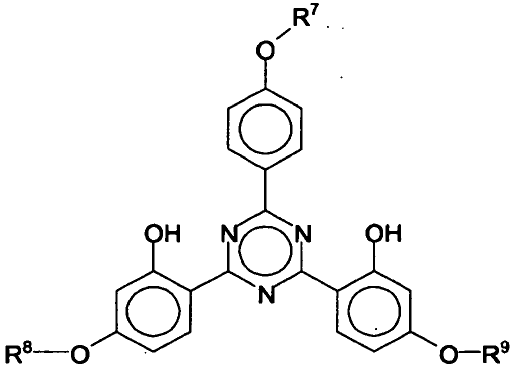 wobei R⁷, R⁸ und R⁹ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl}-1,3,5-triazin (INCI: Aniso Triazin), welches unter der Handelsbezeichnung Tinosorb^{®} S bei der CIBA-Chemikalien GmbH erhältlich ist.

Auch andere UV-Filtersubstanzen, welche das Strukturmotiv 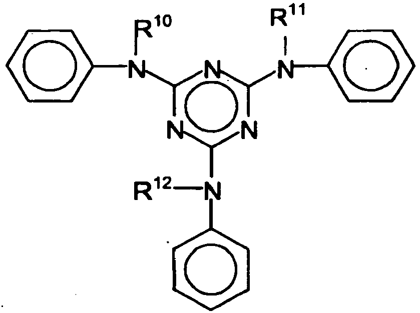 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 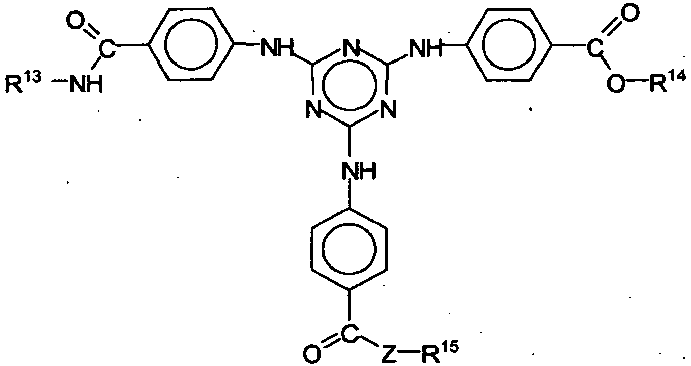 wiedergegeben wird, wobei
- R¹³: einen verzweigten oder unverzweigten C₁-C₁₈-Alkylrest, einen C₅-C₁₂-Cycloalkylrest, gegebenenfalls substituiert mit einer oder mehreren C₁-C₄-Alkylgruppen, darstellt,
- Z: ein Sauerstoffatom oder eine NH-Gruppe darstellt,
- R¹⁴: einen verzweigten oder unverzweigten C₁-C₁₈-Alkylrest, einen C₅-C₁₂-Cycloalkylrest, gegebenenfalls substituiert mit einer oder mehreren C₁-C₄-Alkylgruppen, oder ein Wasserstoffatom, ein Alkalimetallatom, eine Ammoniumgruppe oder eine Gruppe der Formel 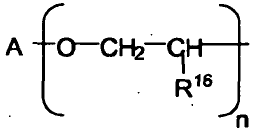 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄-Alkylgruppen,
R¹⁶ ein Wasserstoffatom oder eine Methylgruppe darstellt,
n eine Zahl von 1 bis 10 darstellt,
R¹⁵ einen verzweigten oder unverzweigten C₁-C₁₈-Alkylrest, einen C₅-C₁₂-Cycloalkylrest, gegebenenfalls substituiert mit einer oder mehreren C₁-C₄-Alkylgruppen, darstellt, wenn X die NH-Gruppe darstellt, und einen verzweigten oder unverzweigten C₁-C₁₈-Alkylrest, einen C₅-C₁₂-Cycloalkylrest, gegebenenfalls substituiert mit einer oder mehreren C₁-C₄-Alkylgruppen, oder ein Wasserstoffatom, ein Alkalimetallatom, eine Ammoniumgruppe oder eine Gruppe der Formel 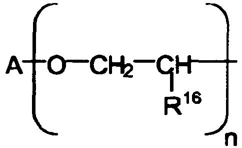 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄-Alkylgruppen,
R¹⁶ ein Wasserstoffatom oder eine Methylgruppe darstellt,
n eine Zahl von 1 bis 10 darstellt,
wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 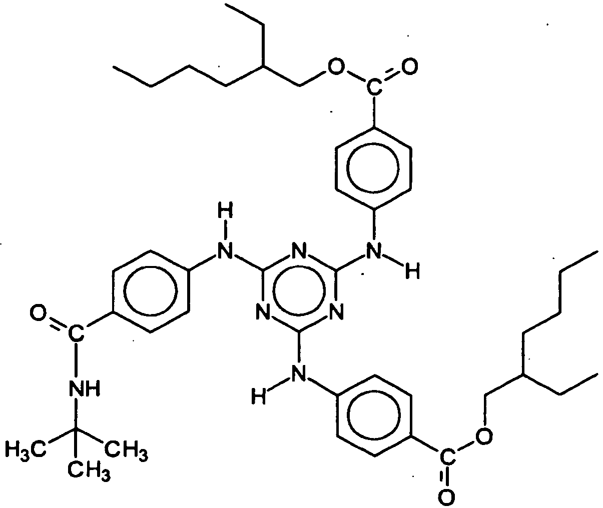 wiedergegeben wird, welches im Folgenden auch als Dioctylbutylamidotriazon (INCI: Diethylhexylbutamidotriazone) bezeichnet wird und unter der Handelsbezeichnung UVASORB^{®} HEB bei Sigma 3V erhältlich ist.

Vorteilhaft im Sinne der vorliegenden Erfindung ist auch ein symmetrisch substituiertes s-Triazin 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL^{®} T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 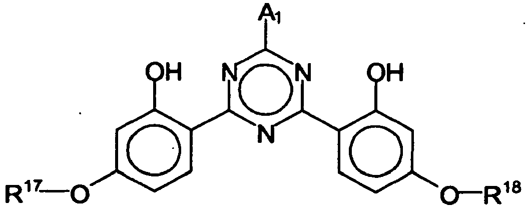 wiedergegeben wird, wobei R¹⁷ und R¹⁸ u. a. C₃-C₁₈-Alkyl oder C₂-C₁₈-Alkenyl und A₁ einen aromatischen Rest repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Vorteilhafte öllösliche UV-B- und/oder Breitband-Filtersubstanzen sind z. B.:
3-Benzylidencampher-Derivate, vorzugsweise 3-(4-Methylbenzyliden)campher, 3-Benzylidencampher;
4-Aminobenzoesäure-Derivate, vorzugsweise 4-(Dimethylamino)-benzoesäure-(2-ethylhexyl)ester, 4-(Dimethylamino)benzoesäureamylester, Derivate des Benzophenons, vorzugsweise 2-Hydroxy-4-methoxybenzophenon (unter der Handelsbezeichnung Uvinul^{®} M40 von der Fa. BASF erhältlich)
   2-Hydroxy-4-methoxy-4'-methylbenzophenon, 2,2'-Dihydroxy-4-methoxybenzophenon, 2,2',4,4'-Tetrahydroxybenzophenon (unter der Handelsbezeichnung Uvinul^{®} D 50 von der Fa. BASF erhältlich).

Besonders vorteilhafte bei Raumtemperatur flüssige UV-Filtersubstanzen im Sinne der vorliegenden Erfindung sind Homomenthylsalicylat,
2-Ethylhexyl-2-cyano-3,3-diphenylacrylat, 2-Ethylhexyl-2-hydroxybenzoat und Ester der Zimtsäure, vorzugsweise 4-Methoxyzimtsäure(2-ethylhexyl)ester und 4-Methoxyzimtsäureisopentylester.

Homomenthylsalicylat (INCI: Homosalate) zeichnet sich durch die folgende Struktur aus:

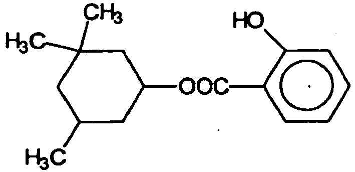

2-Ethylhexyl-2-cyano-3,3-diphenylacrylat (INCI: Octocrylene) ist von BASF unter der Bezeichnung Uvinul^{®} N 539T erhältlich und zeichnet sich durch folgende Struktur aus:

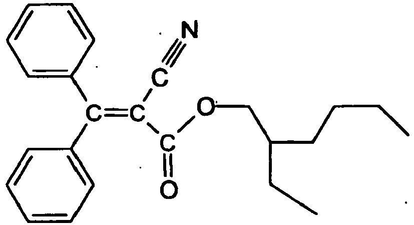

2-Ethylhexyl-2-hydroxybenzoat (2-Ethylhexylsalicylat, Octylsalicylat, INCI: Ethylhexyl Salicylate) ist beispielsweise bei Haarmann & Reimer unter der Handelsbezeichnung Neo Heliopan^{®} OS erhältlich und zeichnet sich durch die folgende Struktur aus:

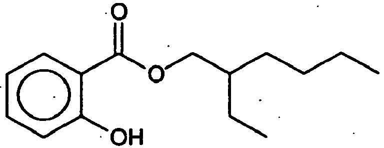

4-Methoxyzimtsäure(2-ethylhexyl)ester (2-Ethylhexyl-4-methoxycinnamat, INCI: Ethylhexyl Methoxycinnamate) ist beispielsweise bei Fa. BASF unter der Handelsbezeichnung Uvinul^{®} MC 80 erhältlich und zeichnet sich durch die folgende Struktur aus:

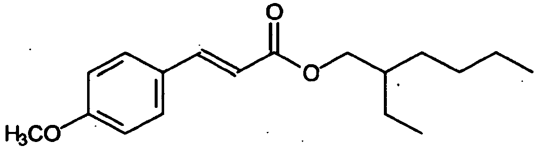

4-Methoxyzimtsäureisopentylester (Isopentyl-4-methoxycinnamat, INCI: Isoamyl p-Methoxycinnamate) ist beispielsweise bei Haarmann & Reimer unter der Handelsbezeichnung Neo Heliopan^{®} E 1000 erhältlich und zeichnet sich durch die folgende Struktur aus:

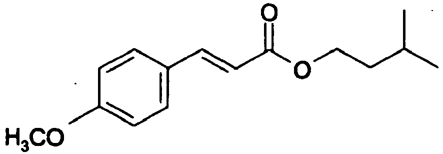

Vorteilhafte Dibenzoylmethanderivate im Sinne der vorliegenden Erfindung sind, insbesondere das 4-(tert.-Butyl)-4'-methoxydibenzoylmethan (CAS-Nr. 70356-09-1), welches von BASF unter der Marke Uvinul^{®} BMBM und von Merck unter der Handelsbezeichnung Eusolex^{®} 9020 verkauft wird zeichnet sich durch folgende Struktur aus:

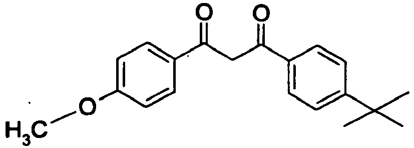

Ein weiteres vorteilhaftes Dibenzoylmethanderivat ist das 4-Isopropyl-Dibenzoylmethan (CAS-Nr. 63250-25-9), welches von Merck unter dem Namen Eusolex^{®} 8020 verkauft wird. Das Eusolex 8020 zeichnet sich durch folgende Struktur aus:

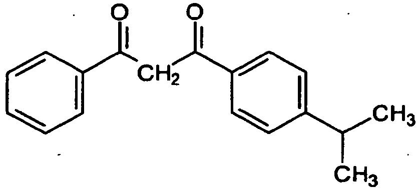

Benzotriazole zeichnen sich durch die folgende Strukturformel aus: 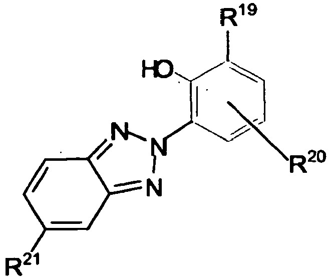 worin
R¹⁹ und R²⁰ unabhängig voneinander lineare oder verzweigte, gesättigte oder ungesättigte, substituierte (z. B. mit einem Phenylrest substituierte) oder unsubstituierte Alkylreste mit 1 bis 18 Kohlenstoffatomen stehen.

Vorteilhaftes Benzotriazol im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]-propyl]-phenol (CAS-Nr.: 155633-54-8) mit der INCI-Bezeichnung Drometrizole Trisiloxane, welches von Fa. Chimex unter der Marke Mexoryl^{®} XL verkauft wird und durch die folgende chemische Strukturformel 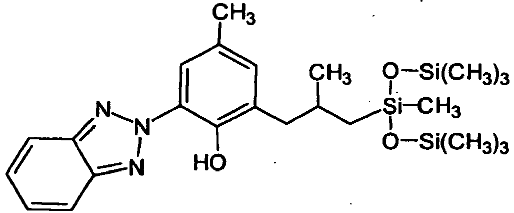 gekennzeichnet ist.

Weitere vorteilhafte Benzotriazole im Sinne der vorliegenden Erfindung sind [2,4'-Dihydroxy-3-(2H-benzotriazol-2-yl)-5-(1,1,3,3-tetramethylbutyl)-2'-n-octoxy-5'-benzoyl]diphenylmethan, 2,2'-Methylen-bis-[6-(2H-benzotriazol-2-yl)-4-(methyl)phenol], 2,2'-Methylen-bis-[6-(2H-benzotriazol-2-yl)-4-(1,1,3,3-tetramethylbutyl)phenol], 2-(2'-Hydroxy-5'-octylphenyl)-benzotriazol, 2-(2'-Hydroxy-3',5'-di-t-amylphenyl)benzotriazol und 2-(2'-Hydroxy-5'-methylphenyl)benzotriazol.

Ein weiterer im Sinne der vorliegenden Erfindung vorteilhafter UV-Filter ist die in EP-A-0 916 335 beschriebene Diphenylbutadienverbindung der folgenden Formel.

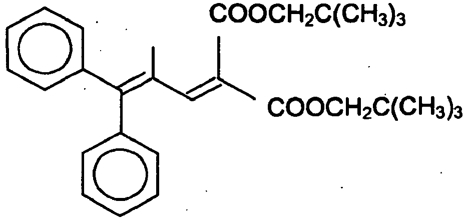

Ein weiterer im Sinne der vorliegenden Erfindung vorteilhafter UV-A-Filter ist der in EP-A-0 895 776 beschriebene 2-(4-Ethoxy-anilinomethylen)-propandicarbonsäure-diethylester der folgenden Formel.

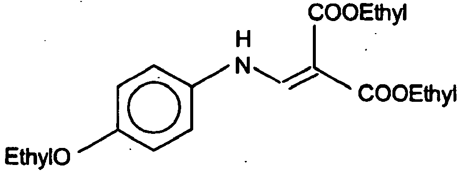

Vorteilhaft im Sinne der vorliegenden Erfindung ist ebenfalls ein aminosubstituiertes Hydroxybenzophenon der folgenden Formel: 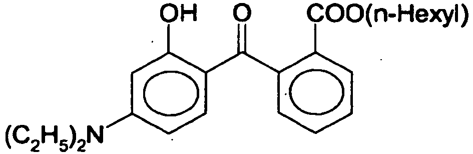 welche von der BASF Aktiengesellschaft als UV-A Filter unter der Warenbezeichnung UVINUL^{®} A Plus vertrieben wird.

Die erfindungsgemäß zu verwendenden Copolymerisate A) eignen sich ebenso für die Verwendung zur Modifizierung der rheologischen Eigenschaften sowie als Solubilisator in pharmazeutischen Zubereitungen jeder Art.

Ein weiterer Gegenstand der Erfindung ist daher ein pharmazeutisches Mittel, enthaltend
A) wenigstens ein stickstoffatomhaltiges hyperverzweigtes Polymer, wie zuvor definiert,
B) wenigstens einen pharmazeutisch akzeptablen Wirkstoff und
C) gegebenenfalls wenigstens einen weiteren, von B) verschiedenen pharmazeutisch akzeptablen Wirk- oder Hilfsstoff.

In einer speziellen Ausführung enthalten die pharmazeutischen Mittel wenigstens einen pharmazeutisch akzeptablen Wirkstoff B), der in Wasser bei 25 °C und 1013 mbar eine Löslichkeit unterhalb 10 g/l aufweist. Dazu dienen die Copolymere A) als Solubilisator für den/die schwerlöslichen Wirkstoff(e). Die Formulierungsgrundlage der erfindungsgemäßen pharmazeutischen Mittel enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB, Ph. Eur., BP, NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, Verdickungsmittel, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung von erfindungsgemäßen pharmazeutischen Mitteln können die Wirkstoffe mit einem geeigneten Hilfsstoff (Exzipient) vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Insbesondere handelt es sich dabei um wässrige Lösungen bzw. Solubilisate zur oralen oder zur parenteralen Applikation. Des Weiteren eignen sich die erfindungsgemäß zu verwendenden Copolymere auch zum Einsatz in oralen Darreichungsformen wie Tabletten, Kapseln, Pulvern, Lösungen. Hier können Sie den schwerlöslichen Arzneistoff mit einer erhöhten Bioverfügbarkeit zur Verfügung stellen. Bei der parenteralen Applikation können neben Solubilisaten auch Emulsionen, beispielsweise Fettemulsionen eingesetzt werden. Auch für diesen Zweck eignen sich die erfindungsgemäßen Copolymere A) um einen schwerlösliche Arzneistoff zu verarbeiten.

Pharmazeutische Formulierungen der oben genannten Art können durch Verarbeiten der erfindungsgemäß zu verwendenden Copolymere A) mit pharmazeutischen Wirkstoffen nach herkömmlichen Methoden und unter Einsatz bekannter und neuer Wirkstoffe erhalten werden.

Die erfindungsgemäße Anwendung kann zusätzlich pharmazeutische Hilfsstoffe und/oder Verdünnungsmittel enthalten. Als Hilfsstoffe werden Cosolventien, Stabilisatoren, Konservierungsmittel besonders aufgeführt.

Die verwendeten pharmazeutischen Wirkstoffe sind in Wasser lösliche oder unlösliche bzw. wenig lösliche Substanzen. Gemäß DAB 9 (Deutsches Arzneimittelbuch) erfolgt die Einstufung der Löslichkeit pharmazeutischer Wirkstoffe wie folgt: wenig löslich (löslich in 30 bis 100 Teilen Lösungsmittel); schwer löslich (löslich in 100 bis 1000 Teilen Lösungsmittel); praktisch unlöslich (löslich in mehr als 10000 Teilen Lösungsmittel). Die Wirkstoffe können dabei aus jedem Indikationsbereich kommen.

Besonders bevorzugt sind von den oben genannten pharmazeutischen Mitteln solche, bei denen es sich um parenteral applizierbare Formulierungen handelt.

Der Gehalt an Copolymer A) in den pharmazeutischen Mitteln liegt, abhängig vom Wirkstoff, im Bereich von 0,01 bis 50 Gew.-%, bevorzugt 0,1 bis 40 Gew.-%, besonders bevorzugt 1 bis 30 Gew.-%, bezogen auf das Gesamtgewicht des Mittels.

Zur Herstellung der erfindungsgemäßen pharmazeutischen Mittel eignen sich prinzipiell alle pharmazeutischen Wirkstoffe und Pro-Drugs. Hierzu zählen Benzodiazepine, Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel. Beispiele für geeignete pharmazeutische Wirkstoffe sind die insbesondere die in den Absätzen 0105 bis 0131 der US 2003/0157170 genannten Wirkstoffe.

Ein weiterer Aspekt der vorliegenden Erfindung betrifft die Verwendung der ampholytischen Copolymere A) als Solubilisatoren in molekulardispersen Systemen. Feststoffdispersionen, also homogene feinstdisperse Phasen von zwei oder mehreren Feststoffen sowie ihr Sonderfall der so genannten "festen Lösungen" (molekulardisperse Systeme), sowie ihr Einsatz in der pharmazeutischen Technologie sind allgemein bekannt (vgl. Chiou und Riegelmann, J. Pharm. Sci., 1971, 60, 1281 - 1300. Daneben betrifft die vorliegenden Erfindung auch feste Lösungen, die mindestens ein erfindungsgemäß zu verwendendes Copolymer A) enthalten.

Die Herstellung von festen Lösungen kann mit Hilfe von Schmelzeverfahren oder nach dem Lösungsverfahren erfolgen.

Als polymerer Hilfsstoff, d. h. Solubilisator für die Herstellung solcher Feststoffdispersionen bzw. fester Lösungen eignen sich die erfindungsgemäßen Copolymere.

Nach dem Schmelzeverfahren können beispielsweise ein Wirkstoff B) und das Copolymer A) im gewünschten Verhältnis, z. B. zu gleichen Teilen abgewogen und gemischt werden. Zur Mischung eignet sich beispielsweise ein Freifallmischer. Die Mischung kann anschließend, z. B. in einem Zweischneckenextruder, extrudiert werden. Der Durchmesser des so erhaltenen, abgekühlten Produktstrangs, bestehend aus einer festen Lösung des gewählten Wirkstoffes in dem gewählten erfindungsgemäß zu verwendenden Copolymeren, ist abhängig vom Durchmesser der Perforation der Lochscheiben des Extruders. Durch das Abschneiden der gekühlten Produktstränge mit Hilfe eines rotierenden Messers können zylindrische Partikel gewonnen werden, deren Höhe abhängig ist vom Abstand zwischen Lochscheibe und Messer. Der mittlere Durchmesser der zylindrischen Partikel beträgt in der Regel etwa 1000 bis etwa 3000 µm, die Höhe in der Regel etwa 2000 bis etwa 5000 µm. Größere Extrudate können in einem nachgeschalteten Schritt zerkleinert werden.

Alternativ kann man feste Lösung auch im Lösungsverfahren herstellen. Hierzu löst man üblicherweise den Wirkstoff B) und das Copolymer A) in einem geeigneten Lösungsmittel. Anschließend wird die Lösung üblicherweise in eine geeignete Form gegossen, und das Lösungsmittel, beispielsweise durch Trocknung, entfernt. Die Trocknungsbedingungen wählt man vorteilhaft je nach den Eigenschaften von Wirkstoff (z. B Thermolabilität) und Lösungsmittel (z. B. Siedepunkt).

Unter Beachtung des Materialverhaltens kann der entstandene Formling bzw. das Extrudat beispielsweise mit einer geeigneten Mühle (z. B. Stiftmühle) zerkleinert werden. Die feste Lösung zerkleinert man vorteilhafterweise bis zu einer mittleren Teilchengröße von weniger als etwa 2000 µm, bevorzugt weniger als etwa 1000 µm und besonders bevorzugt weniger als etwa 500 µm.

Mit geeigneten Hilfsstoffen kann nun das entstandene Schüttgut zu einer Tablettiermischung oder zu einem Kapselfüllgut verarbeitet werden. Die Tablettierung führt man vorteilhaft so durch, dass man Tabletten mit Härte von größer etwa 35 N, bevorzugt größer etwa 60 N, besonders bevorzugt von etwa 80 bis etwa 100 N erhält.

Die so erhältlichen Formulierungen können wie herkömmliche Formulierungen erforderlichenfalls mit geeigneten Überzugsmaterialien zur Erzielung von Magensaftresistenz, Retardierung, Geschmacksmaskierung usw. überzogen werden.

Neben der Anwendung in der Pharmazie eignen sich die erfindungsgemäß zu verwendenden Copolymere A) auch im Lebensmittelbereich zur Modifizierung der rheologischen Eigenschaften und/oder als Solubilisatoren für schwer wasserlösliche oder wasserunlösliche Nähr-, Hilfs- oder Zusatzstoffe, wie z. B. fettlösliche Vitamine oder Carotinoide. Als Beispiele seien klare, mit Carotinoiden gefärbte Getränke genannt. Gegenstand der Efindung sind daher auch lebensmitteltechnische Zubereitungen, die mindestens eines der erfindungsgemäß zu verwendenden Copolymere A) enthalten. Zu den Lebensmittelzubereitungen sind im Rahmen der vorliegenden Erfindung auch Nahrungsergänzungsmittel wie z. B. Lebensmittelfarbstoffe enthaltende Zubereitungen und diätetische Lebensmittel zu verstehen. Darüber hinaus eigenen sich die genannten Copolymere A) auch zur Modifizierung der rheologischen Eigenschaften und/oder als Solubilisatoren für Futtermittelzusätze für die Tierernährung.

Außerdem eignen sich die ampholytischen Copolymere A) zur Herstellung wässriger Zubereitungen von Nahrungsergänzungsmitteln wie wasserunlöslichen Vitaminen und Provitaminen wie Vitamin A, Vitamin A-Acetat, Vitamin D, Vitamin E, Tocopherol-Derivate wie Tocopherolacetat und Vitamin K.

Beispiele für Effektstoffe, die als erfindungsgemäße wässrige Wirkstoffzusammensetzung formuliert werden können, sind Farbstoffe: z. B. die in DE-A 102 45 209 beschriebenen Farbstoffe sowie die gemäß Colour-Index als Disperse-Farbstoffe und als Solvent-Farbstoffe bezeichneten Verbindungen, die auch als Dispersionsfarbstoffe bezeichnet werden. Eine Zusammenstellung geeigneter Dispersionsfarbstoffe findet sich beispielsweise in Ullmanns Enzyklopädie der technischen Chemie, 4. Auflage, Bd. 10, S. 155-165 (siehe auch Bd. 7, S. 585ff - Anthrachinonfarbstoffe; Bd. 8, S. 244ff-Azofarbstoffe; Bd. 9, S. 313ff - Chinophthalonfarbstoffe). Auf diese Literaturstelle und die darin genannten Verbindungen wird hiermit ausdrücklich Bezug genommen. Erfindungsgemäß geeignete Dispersionsfarbstoffe und Solvent-Farbstoffe umfassen verschiedenste Farbstoffklassen mit unterschiedlichen Chromophoren, beispielsweise Anthrachinonfarbstoffe, Monoazo- und Disazofarbstoffe, Chinophthalone, Methin- und Azamethinfarbstoffe, Naphthalimidfarbstoffe, Naphthochinonfarbstoffe und Nitrofarbstoffe. Beispiele für erfindungsgemäß geeignete Dispersionsfarbstoffe sind die Dispersionsfarbstoffe der folgenden Colour-Index Liste: C. I. Disperse Yellow 1 - 228, C. I. Disperse Orange 1 - 148, C. I. Disperse Red 1 - 349, C. I. Disperse Violet 1 - 97, C. I. Disperse Blue 1 - 349, C. I. Disperse Green 1 - 9, C. I. Disperse Brown 1 - 21, C. I. Disperse Black 1 - 36. Beispiele für erfindungsgemäß geeignete Solvent-Farbstoffe sind die Verbindungen der folgenden Colour-Index Liste: C. I. Solvent Yellow 2 - 191, C. I. Solvent Orange 1 - 113, C. I. Solvent Red 1 - 248, C. I. Solvent Violet 2 - 61, C. I. Solvent Blue 2 - 143, C. I. Solvent Green 1 - 35, C. I. Solvent Brown 1 - 63, C. I. Solvent Black 3 - 50. Erfindungsgemäß geeignete Farbstoffe sind weiterhin Derivate des Naphthalins, des Anthracens, des Perylens, des Terylens, des Quarterylens, sowie Diketopyrrolopyrrolfarbstoffe, Perinonfarbstoffe, Cumarinfarbstoffe, Isoindolin- und Isoindolinonfarbstoffe, Porphyrinfarbstoffe, Phthalocyanin- und Naphthalocyaninfarbstoffe.

Neben den vorgenannten Bestandteilen können die erfindungsgemäßen Wirk- und Effektstoffzusammensetzungen auch konventionelle oberflächenaktive Substanzen und sonstige Additive enthalten. Zu den oberflächenaktiven Substanzen zählen Tenside, Dispergierhilfsmittel und Netzmittel. Zu den sonstigen Additiven zählen insbesondere Verdickungsmittel, Entschäumer, Konservierungsmittel, Frostschutzmittel Stabilisierungsmittel, etc.

Prinzipiell brauchbar sind anionische, kationische, nichtionische und amphotere Tenside, wobei Polymertenside sowie Tenside mit Heteroatomen in der hydrophoben Gruppe eingeschlossen sind.

Zu den anionischen Tensiden gehören beispielsweise Carboxylate, insbesondere Alkali-, Erdalkali- und Ammoniumsalze von Fettsäuren, z. B. Kaliumstearat, die üblicherweise auch als Seifen bezeichnet werden; Acylglutamate; Sarkosinate, z. B. Natriumlauroylsarkosinat; Taurate; Methylcellulosen; Alkylphosphate, insbesondere Mono- und Diphosphorsäurealkylester; Sulfate, insbesondere Alkylsulfate und Alkylethersulfate; Sulfonate, weitere Alkyl- und Alkylarylsulfonate, insbesondere Alkali-, Erdalkali- und Ammoniumsalze von Arylsulfonsäuren sowie alkylsubstituierten Arylsulfonsäuren, Alkylbenzolsulfonsäuren, wie beispielsweise Lignin- und Phenolsulfonsäure, Naphthalin-und Dibutylnaphthalinsulfonsäuren, oder Dodecylbenzolsulfonate, Alkylnaphthalinsulfonate, Alkylmethylestersulfonate, Kondensationsprodukte von sulfoniertem Naphthalin und Derivaten davon mit Formaldehyd, Kondensationsprodukte von Naphthalinsulfonsäuren, Phenol- und/oder Phenolsulfonsäuren mit Formaldehyd oder mit Formaldehyd und Harnstoff, Mono- oder Dialkylbemsteinsäureestersulfonate; sowie Eiweißhydrolysate und Lignin-Sulfitablaugen. Die zuvor genannten Sulfonsäuren werden vorteilhafterweise in Form ihrer neutralen oder gegebenenfalls basischen Salze verwendet.

Zu den kationischen Tensiden gehören beispielsweise quaternierte Ammoniumverbindungen, insbesondere Alkyltrimethylammonium- und Dialkyldimethylammonium-Halogenide und -alkylsulfate sowie Pyridin- und Imidazolin-Derivate, insbesondere Alkylpyridinium-Halogenide.

Zu den nichtionischen Tensiden gehören beispielsweise:
- Fettalkoholpolyoxyethylenester, beispielsweise Laurylalkoholpofyoxyethylenetheracetat,
- Alkyl-Polyoxyethylen- und -polyoxypropylenether, z. B. von iso-Tridecylalkohol und Fettalkohol-Polyoxyethylenether,
- Alkylarylalkohol-Polyoxyethylenether, z. B. Octylphenol-Polyoxyethylenether,
- alkoxylierte tierische und/oder pflanzliche Fette und/oder Öle, beispielsweise Maisölethoxylate, Rizinusölethoxylate, Talgfettethoxylate,
- Glycerinester, wie beispielsweise Glycerinmonostearat,
- Fettalkoholalkoxylate und Oxoalkoholalkoxylate, insbesondere vom Typ RO-(R₁₈O)ᵣ(R₁₉O)ₛR₂₀ mit R₁₈ und R₁₉ unabhängig voneinander = C₂H₄, C₃H₆, C₄H₈ und R₂₀ = H, oder C₁-C₁₂-Alkyl, R = C₃-C₃₀-Alkyl oder C₆-C₃₀-Alkenyl, r und s unabhängig voneinander 0 bis 50, wobei nicht beide für 0 stehen können, wie iso-Tridecylalkohol und Oleylalkoholpolyoxyethylenether,
- Alkylphenolalkoxylate, wie beispielsweise ethoxyliertes iso-Octyl-, Octyl- oder Nonylphenol, Tributylphenolpolyoxyethylenether,
- Fettaminalkoxylate, Fettsäureamid- und Fettsäurediethanolamidalkoxylate, insbesondere deren Ethoxylate,
- Zuckertenside, Sorbitester, wie beispielsweise Sorbitanfettsäureester (Sorbitanmonooleat, Sorbitantristearat), Polyoxyethylensorbitanfettsäureester, Alkylpolyglycoside, N-Alkylgluconamide,
- Alkylmethylsulfoxide,
- Alkyldimethylphosphinoxide, wie beispielsweise Tetradecyldimethylphosphinoxid.

Zu den amphoteren Tensiden gehören beispielsweise Sulfobetaine, Carboxybetaine und Alkyldimethylaminoxide, z. B. Tetradecyldimethylaminoxid.

Weitere Tenside, die hier beispielhaft genannt werden sollen, sind Perfluortenside, Silikontenside, Phospholipide, wie beispielsweise Lecithin oder chemisch modifizierte Lecithine, Aminosäuretenside, z. B. N-Lauroylglutamat.

Sofern nicht spezifiziert, handelt es sich bei den Alkylketten der oben aufgeführten Tenside um lineare oder verzweigte Reste mit üblicherweise 8 bis 20 Kohlenstoffatomen.

In einer Ausführungsform enthalten die erfindungsgemäßen wässrigen Wirkstoffzusammensetzung nicht mehr als 10 Gew.%, vorzugsweise nicht mehr als 5 Gew.-% und insbesondere nicht mehr als 3 Gew.%, z. B. 0,01 bis 5 Gew.% oder 0,1 bis 3 Gew.-% an konventionellen oberflächenaktiven Substanzen, jeweils bezogen auf die Gesamtmenge an Wirkstoff und Polymerzusammensetzung. Die konventionellen oberflächenaktiven Substanzen machen dann vorzugsweise nicht mehr als 5 Gew.% und insbesondere nicht mehr als 3 Gew.%, z. B. 0,01 bis 5 Gew.% oder 0,1 bis 3 Gew.-%, bezogen auf das Gesamtgewicht der Zusammensetzung aus.

Je nach Anwendung kann es jedoch von Vorteil sein, wenn die erfindungsgemäßen Wirkstoffzusammensetzungen mit oberflächenaktiven Substanzen formuliert werden. Dann liegt der Anteil an konventioneller oberflächenaktiver Substanz häufig im Bereich von 0,5 bis 30 Gew.%, insbesondere im Bereich von 1 bis 20 Gew.-%, bezogen auf die Gesamtmenge an Wirkstoff und Polymerzusammensetzung, bzw. im Bereich von 0,2 bis 20 Gew.-% und insbesondere im Bereich von 0,5 bis 15 Gew.%, bezogen auf das Gesamtgewicht der formulierten Zusammensetzung.

Auch wenn ein Vorteil der erfindungsgemäßen Zusammensetzungen ihr geringer Gehalt an flüchtigen organischen Substanzen ist, kann es für einige Anwendungen erwünscht sein, die erfindungsgemäßen Zusammensetzungen mit organischen Lösungsmitteln, Ölen und Fetten, vorzugsweise solchen Lösungsmitteln oder Ölen und Fetten, die umweltverträglich oder biokompatibel sind zu versetzen, z. B. die vorgenannten mit Wasser mischbaren Lösungsmittel oder Lösungsmittel, Ölen oder Fetten die mit Wasser nicht oder nur sehr begrenzt mischbar sind, z. B.:
- Paraffinöle, aromatische Kohlenwasserstoffe und aromatische Kohlenwasserstoffgemische, z. B. Xylole, Solvesso 100, 150 oder 200, und dergleichen,
- Phenole und Alkylphenole, z. B. Phenol, Hydrochinon, Nonylphenol, etc.
- Ketone mit mehr als 4 C-Atomen wie Cyclohexanon, Isophoron, Isopheron, Acetophenon, Acetonaphthon,
- Alkohole mit mehr als 4 C-Atomen wie acetylierter Lanolinalkohol, Cetylalkohol, 1-Decanol, 1-Heptanol, 1-Hexanol, Isooctadecanol, Isopropylalkohol, Oleylalkohol, Benzylalkohol,
- Carbonsäureester, z. B. Adipinsäuredialkylester wie Adipinsäurebis(2-ethylhexyl)ester, Phthalsäuredialkylester wie Phthalsäurebis(2-ethylhexyl)ester, Essigsäurealkylester (auch verzweigte Alkylgruppen) wie Ethylacetat und Acetoessigsäureethylester, Stearate wie Butylstearat, Glycerinmonostearat, Citrate wie Acetyltributylcitrat, weiterhin Cetyloctanoat, Methyloleat, Methyl-p-hydroxybenzoat, Methyltetradecanoat, Propyl-p-hydroxybenzoat, Methylbenzoat, Milchsäureester wir Isopropyllacttat, Butyllactat und 2-Ethylhexyllactat,
- Pflanzenöle wie Palmöl, Rapsöl, Rizinusöl und Derivate davon wie z. B. oxidiert, Kokusnussöl, Lebertran, Maiskeimöl, Sojabohnenöl, Leinsamenöl, Olivenöl, Erdnussöl, Färberdistelöl, Sesamsamenöl, Grapefruitöl, Basilikumöl, Aprikosenöl, Ingweröl, Geranienöl, Orangenöl, Rosmarinöl, Macadamiaöl, Zwiebelöl, Mandarinenöl, Kiefernöl, Sonnenblumenöl,
- hydrogenierte Pflanzenöle wie hydrogeniertes Palmöl, hydrogeniertes Rapsöl, hydrogeniertes Sojabohnenöl,
- tierische Öle wie Schweinefettöl, Fischöle,
- Dialkylamide mittel bis langkettiger Fettsäuren z. B. Halicomide sowie
- Pflanzenölester wie Rapsölmethylester.

Die Copolymere A) können gemeinsam mit konventionellen Verdickem eingesetzt werden.

Geeignete Verdicker sind Verbindungen, die der Formulierung ein pseudoplastisches Fließverhalten verleihen, d.h. hohe Viskosität im Ruhezustand und niedrige Viskosität im bewegten Zustand. Hier sind beispielsweise Polysaccharide bzw. organische Schichtmineralien wie Xanthan Gum^{®} (Kelzan^{®} der Fa. Kelco), Rhodopol^{®} 23 (Rhone Poulenc) oder Veegum^{®} (Firma R. T. Vanderbilt) oder Attaclay^{®} (Firma Engelhardt) zu nennen, wobei Xanthan-Gum^{®} bevorzugt verwendet wird.

Als für erfindungsgemäße Dispersionen geeignete Antischaummittel kommen beispielsweise Silikonemulsionen (wie z. B. Silikon^{®} SRE, Firma Wacker oder Rhodorsil^{®} der Firma Rhodia), langkettige Alkohole, Fettsäuren, fluororganische Verbindungen und deren Gemische in Betracht.

Bakterizide können zur Stabilisierung den erfindungsgemäßen Zusammensetzungen gegen Befall mit Mikroorganismen zugesetzt werden. Geeignete Bakterizide sind beispielsweise Proxel^{®} der Fa. ICI oder Acticide^{®} RS der Fa. Thor Chemie und Kathon^{®} MK der Firma Rohm & Haas.

Geeignete Frostschutzmittel sind organische Polyole, z. B. Ethylenglycol, Propylenglycol oder Glycerin. Diese werden üblicherweise in Mengen von nicht mehr als 10 Gew.-%, bezogen auf das Gesamtgewicht der Wirkstoffzusammensetzung eingesetzt, um den gewünschten Gehalt an flüchtigen Verbindungen nicht zu überschreiten. In einer Ausführungsform der Erfindung beträgt der Anteil hiervon verschiedener flüchtiger organischer Verbindungen vorzugsweise nicht mehr als 1 Gew.%, insbesondere nicht mehr als 1000 ppm.

Gegebenenfalls können die erfindungsgemäßen Wirkstoffzusammensetzungen 1 bis 5 Gew.-% Puffer bezogen auf die Gesamtmenge der hergestellten Formulierung zur pH-Wert Regulation enthalten, wobei sich die Menge und Art des eingesetzten Puffers nach den chemischen Eigenschaften des Wirkstoffes bzw. der Wirkstoffe richtet. Beispiele für Puffer sind Alkalisalze schwacher anorganischer oder organischer Säuren wie z. B. Phosphorsäure, Borsäure, Essigsäure, Propionsäure, Citronensäure, Fumarsäure, Weinsäure, Oxalsäure und Bernsteinsäure.

In einer besonders bevorzugten Ausführungsform werden die erfindungsgemäßen Copolymere als Komponente in einem kosmetischen Mittel eingesetzt. Sie können dabei, wie zuvor beschrieben, zur Modifizierung der rheologischen Eigenschaften eines kosmetischen Mittels auf Basis eines wässrigen Mediums dienen. Sie können weiterhin als Solubilisatoren für kosmetische Mittel dienen, die wenigstens einen kosmetisch akzeptablen Wirk- oder Effektstoff enthalten, der in Wasser bei 25 °C und 1013 mbar eine Löslichkeit unter 10 g/l aufweist. Unabhängig davon verfügen die erfindungsgemäßen Copolymere A) auch über gute Filmbildungseigenschaften und können als solche auch als kosmetischer Wirkstoff eingesetzt werden.

Ein weiterer Gegenstand der Erfindung ist ein kosmetisches oder pharmazeutisches Mittel, enthaltend
α) wenigstens ein ampholytisches Copolymer A), wie zuvor definiert, und
β) wenigstens einen kosmetisch oder pharmazeutisch akzeptablen Träger.

Vorzugsweise ist die Komponente β) ausgewählt unter
i) Wasser,
ii) wassermischbaren organischen Lösungsmitteln, vorzugsweise C₂-C₄-Alkanolen, insbesondere Ethanol,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen,
und Mischungen davon.

Geeignete hydrophile und hydrophobe Komponenten β) sind die zuvor genannten.

Speziell geeignete kosmetisch verträgliche Öl- bzw. Fettkomponenten β) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Bevorzugte hydrophile Träger β) sind ausgewählt unter Wasser, 1-, 2- oder mehrwertigen Alkoholen mit vorzugsweise 1 bis 8 Kohlenstoffatomen, wie Ethanol, n-Propanol, iso-Propanol, Propylenglykol, Glycerin, Sorbit, etc.

Bei den erfindungsgemäßen kosmetischen Mitteln kann es sich um hautkosmetische, haarkosmetische, dermatologische, hygienische oder pharmazeutische Mittel handeln.

Aufgrund ihrer filmbildenden und verdickenden Eigenschaften eignen sich die zuvor beschriebenen Copolymere A) insbesondere als Zusatzstoffe für Haar- und Hautkosmetika. Sie eignen sich speziell zur Formulierung von Gelen.

Vorzugsweise liegen die erfindungsgemäßen Mittel in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden.

Die erfindungsgemäßen kosmetisch aktiven Mittel können zusätzlich kosmetisch und/oder dermatologisch aktive Wirk- und Eftektstoffe sowie Hilfsstoffe enthalten. Geeignet sind prinzipiell die zuvor genannten Wirk- und Effektstoffe B) sowie Hilfsstoffe C). In einer speziellen Ausführung enthalten die erfindungsgemäßen kosmetischen Mittel wenigstens einen wasserunlöslichen oder nur gering wasserlöslichen Wirk- oder Effektstoff.

Vorzugsweise enthalten die erfindungsgemäßen kosmetischen Mittel wenigstens ein wie vorstehend definiertes Copolymer A), wenigstens einen wie vorstehend definierten Träger β) und wenigstens einen davon verschiedenen Bestandteil, der vorzugsweise ausgewählt ist unter kosmetisch aktiven Wirkstoffen, Emulgatoren, Tensiden, Konservierungsmitteln, Parfümölen, zusätzlichen Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Tönungsmitteln, Bräunungsmitteln, Farbstoffen, Pigmenten, Konsistenzgebern, Feuchthaltemitteln, Rückfettem, Collagen, Eiweißhydrolysaten, Lipiden, Antioxidantien, Entschäumern, Antistatika, Emollienzien und Weichmachern.

Zusätzlich zu den Copolymeren A) geeignete konventionelle Verdickungsmittel in derartigen Formulierungen sind vernetzte Polyacrylsäuren und deren Derivate, Polysaccharide und deren Derivate, wie Xanthan-gum, Agar-Agar, Alginate oder Tylosen, Cellulosederivate, z. B. Carboxymethylcellulose oder Hydroxycarboxymethylcellulose; Fettalkohole, Monoglyceride und Fettsäuren, Polyvinylalkohol und Polyvinylpyrrolidon.

Geeignete kosmetisch und/oder dermatologisch aktive Wirkstoffe sind z. B. Haut- und Haarpigmentierungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, desodorierende Wirkstoffe, sebostatische Wirkstoffe, Pflanzenextrakte, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z. B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-B- und/oder UV-A-Bereich absorbieren. Geeignete UV-Filter sind die zuvor genannten. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc. Geeignete hyperemisierend wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Die erfindungsgemäßen kosmetischen Mittel können als kosmetischen und/oder pharmazeutischen Wirkstoff (wie auch gegebenenfalls als Hilfsstoff) wenigstens ein kosmetisch oder pharmazeutisch akzeptables Polymer enthalten, das sich von den erfindungsgemäßen Copolymeren A) unterscheidet. Dazu zählen ganz allgemein anionische, kationische, amphotere und neutrale Polymere. Auf die zuvor genannten Polymere wird hier voll Bezug genommen.

Nach einer bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Geeignete hautkosmetische Mittel sind z. B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyelinern, Rouges, Puder und Augenbrauenstifte.

Außerdem können die Copolymere A) verwendet werden in Nose-Strips zur Porenreinigung, in Antiaknemitteln, Repellents, Rasiermitteln, Haarentfernungsmitteln, Intimpflegemitteln, Fußpflegemitteln sowie in der Babypflege.

Bei den erfindungsgemäßen Hautpflegemitteln handelt es sich insbesondere um W/O-oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen Copolymere A) zeigen vorteilhafte Wirkungen. Die Polymere können unter anderem zur Feuchthaltung und Konditionierung der Haut und zur Verbesserung des Hautgefühls beitragen. Durch Zusatz der erfindungsgemäßen Polymere kann in bestimmten Formulierungen eine erhebliche Verbesserung der Hautverträglichkeit erreicht werden.

Hautkosmetische und dermatologische Mittel enthalten vorzugsweise wenigstens ein Copolymere A) in einem Anteil von etwa 0,001 bis 30 Gew.-%, vorzugsweise 0,01 bis 20 Gew.-%, ganz besonders bevorzugt 0,1 bis 12 Gew.%, bezogen auf das Gesamtgewicht des Mittels.

Besonders Lichtschutzmittel auf Basis der Copolymere A) besitzen die Eigenschaft, die Verweilzeit der UV-absorbierenden Inhattsstoffe im Vergleich zu gängigen Hilfsmitteln wie Polyvinylpyrrolidon zu erhöhen.

Je nach Anwendungsgebiet können die erfindungsgemäßen Mittel in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den Copolymere A) und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z. B. Paraffine, Siliconöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von C₆-C₃₀-Fettsäuren, Wachsester, wie z. B. Jojobaöl, Fettalkohole, Vaseline, hydriertes Lanolin und acetyliertes Lanolin sowie Mischungen davon.

Man kann die erfindungsgemäßen Polymere auch mit herkömmlichen Polymeren, wie zuvor beschrieben, abmischen, falls spezielle Eigenschaften eingestellt werden sollen.

Zur Einstellung bestimmter Eigenschaften wie z. B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen, insbesondere als Wasser-in-Öl-(W/O)- oder Öl-in-Wasser(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben wenigstens einem Copolymere A) in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z. B. für eine Hautcreme etc., enthält im Allgemeinen eine wässrige Phase, die mittels eines geeigneten Emulgatorsystems in einer Öl- oder Fettphase emulgiert ist. Zur Bereitstellung der wässrigen Phase kann ein Copolymer A) eingesetzt werden.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250 °C und deren Destillationsendpunkt bei 410 °C liegt, wie z. B. Vaselinöl; Ester gesättigter oder ungesättigter Fettsäuren, wie Alkylmyristate, z. B. i-Propyl-, Butyl- oder Cetylmyristat, Hexadecylstearat, Ethyl-oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Neben den Copolymeren A) können auch Wachse verwendet werden, wie z. B. Carnaubawachs, Candilillawachs, Bienenwachs, mikrokristallines Wachs, Özokeritwachs und Ca-, Mg- und Al-Oleate, -Myristate, -Linoleate und -Stearate.

Weiterhin kann eine erfindungsgemäße Emulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens ein Copolymer A) sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säure, Konservierungsstoffen und Antioxidantien sowie Verdickern/Gelbildnern, Hautkondkioniermitteln und Feuchthaltemitteln.

Diese Formulierungen enthalten vorzugsweise 2 bis 50 Gew.%, bevorzugt 5 bis 40 Gew.%, besonders bevorzugt 8 bis 30 Gew.% Tenside, bezogen auf das Gesamtgewicht der Formulierung.

In den Wasch-, Dusch- und Badepräparaten können alle in Körperreinigungsmitteln üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten im Molekül aufweisen.

Dazu zählen z. B. Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglycoside oder Sorbitanetherester geeignet.

Außerdem können die Wasch-, Dusch- und Badepräparate übliche kationische Tenside enthalten, wie z. B. quaternäre Ammoniumverbindungen, beispielsweise Cetyltrimethylammoniumchlorid.

Weiterhin können die Duschgel-/Shampoo-Formulierungen Verdicker, wie z. B. Kochsalz, PEG-55, Propylenglykol-Oleat, PEG-120-Methylglucosedioleat und andere, sowie Konservierungsmittel, weitere Wirk- und Hilfsstoffe und Wasser enthalten.

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens ein Copolymer A) in einer Menge im Bereich von etwa 0,1 bis 30 Gew.%, bevorzugt 0,5 bis 20 Gew.-%, bezogen auf das Gesamtgewicht des Mittels.

Vorzugsweise liegen die erfindungsgemäßen Haarbehandlungsmittel in Form eines Schaumfestigers, Haarmousses, Haargels, Shampoos, Haarsprays, Haarschaums, Spitzenfluids, Egalisierungsmittels für Dauerwellen oder "Hot-Oil- Treatments" vor. Je nach Anwendungsgebiet können die haarkosmetischen Zubereitungen als (Aerosol-)Spray, (Aerosol-)Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Die erfindungsgemäßen haarkosmetischen Formulierungen enthalten in einer bevorzugten Ausführungsform
a) 0,05 bis 20 Gew.% wenigstens eines Copolymers A),
b) 20 bis 99,95 Gew.% Wasser und/oder Alkohol,
c) 0 bis 50 Gew.% wenigstens eines Treibgases,
d) 0 bis 5 Gew.-% wenigstens eines Emulgators,
e) 0 bis 3 Gew.% wenigstens eines Verdickers, sowie
f) bis zu 25 Gew.% weitere Bestandteile.

Unter Alkohol sind alle in der Kosmetik üblichen Alkohole zu verstehen, z. B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d. h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z. B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Stabilisatoren, pH-Wert-Regulatoren, Farbstoffe, Viskositätsregulierer, Gelbildner, Salze, Feuchthaltemittel, Rückfetter, Komplexbildner und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Als herkömmliche Haarkosmetik-Polymere eignen sich beispielsweise die zuvor genannten kationischen, anionischen, neutralen, nichtionischen und amphoteren Polymere, auf die hier Bezug genommen wird.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Polymerisate eignen sich insbesondere als Festigungsmittel in Haarstyling-Zubereitungen, insbesondere Haarschäume.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z. B. Laureth-4; Cetethe, z. B. Ceteth-1, Polyethylenglykolcetylether; Cetearethe, z. B. Ceteareth-25, Polyglykolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammonium-dihydrogenphosphat, Cetyltrimoniumchlorid, Cetyltrimmoniumbromid, Cocotrimoniummethylsulfat, Quatemium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder PropylenoxidEinheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:
a) 0,1 bis 5 Gew.-% wenigstens eines Copolymers A),
b) 0 bis 5 Gew.% wenigstens eines von A) verschiedenen kosmetisch akzeptablen wasserlöslichen oder wasserdispergierbaren Haarfestigerpolymers,
c) 80 bis 99,85 Gew.% Wasser und/oder Alkohol,
d) 0 bis 1 Gew.-% eines von A) verschiedenen Gelbildners,
e) 0 bis 20 Gew.% weitere Bestandteile.

Als zusätzliche Gelbildner können alle in der Kosmetik üblichen Gelbildner eingesetzt werden. Hierzu zählen leicht vernetzte Polyacrylsäure, beispielsweise Carbomer (INCI), Cellulosederivate, z. B. Hydroxypropylcellulose, Hydroxyethylcellulose, kationisch modifizierte Cellulosen, Polysaccharide, z. B. Xanthangummi, Capryl/Caprin-Triglycerid, Natriumacrylat-Copolymere, Polyquatemium-32 (und) Paraffinum Liquidum (INCI), Natriumacrylat-Copolymere (und) Paraffinum Liquidum (und) PPG-1 Trideceth-6, Acrylamidopropyltrimoniumchlorid/Acrylamid-Copolymere, Steareth-10 Allylether Acrylat-Copolymere, Polyquaternium-37 (und) Paraffinum Liquidum (und) PPG-1 Trideceth-6, Polyquatemium 37 (und) Propylenglycoldicapratdicaprylat (und) PPG-1 Trideceth-6, Polyquaternium-7, Polyquaternium-44. Als zusätzliche Gelbildner geeignete vernetzte Homopolymere der Acrylsäure sind beispielsweise kommerziell unter dem Namen Carbopol® von der Firma BF GOODRICH erhältlich. Bevorzugt sind auch hydrophob modifizierte vernetzte Polyacrylat-Polymere, wie Carbopol® Ultrez 21 von der Firma Noveon. Weitere Beispiele für als Gelbildner geeignete anionische Polymere sind Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Besonders geeignete Polymere sind Copolymere aus (Meth)acrylsäure und Polyetheracrylaten, wobei die Polyetherkette mit einem C₈-C₃₀-Alkylrest terminiert ist. Dazu zählen z. B. Acrylat/Beheneth-25-methacrylat-Copolymere, die unter der Bezeichnung Aculyn® von der Firma Rohm und Haas erhältlich sind.

Die erfindungsgemäßen Copolymere A) können in kosmetischen Zubereitungen als Konditioniermittel eingesetzt werden.

Geeignete anionische Tenside zur Formulierung mit den Copolymeren A)sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanofamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z. B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den Copolymeren A) eingesetzt werden. Hierzu zählen beispielsweise die zuvor genannten kationischen Polymere mit der Bezeichnung Polyquatemium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Ein weiterer Gegenstand der Erfindung ist die Verwendung eines Copolymers A), wie zuvor definiert, als Hilfsmittel in der Pharmazie, bevorzugt als oder in Beschichtungsmittel(n) für feste Arzneiformen, zur Modifizierung rheologischer Eigenschaften, als oberflächenaktive Verbindung, als oder in Klebemittel(n) sowie als oder in Beschichtungsmittel(n) für die Textil-, Papier-, Druck- und Lederindustrie.

Die Erfindung wird anhand der folgenden nicht einschränkenden Beispiele näher erläutert.

### Beispiele :

### Allgemeine Herstellungsvorschrift A1:

### Beispiel 16: Copolymer aus VP/VI-MAS/MAS/MMA/C₁₈-PEG-MA/EGDMA

| | | |
|---|---|---|
| Vorlage: | 412 g | Butylacetat |
| Zulauf 1: | 58,5 g | Vinylpyrrolidon |
| | 18,0 g | Vinylimidazol;Methacrylsäure [1:1] |
| | 57,0 g | Methacrylsäure |
| | 15,0 g | Plex-6877 O® (25%ige C₁₈-PEG-MA in Methylmethacrylat) |
| | 1,5 g | Ethylenglykoldimethacrylat |
| Zulauf 2: | 38,2 g | Butylacetat |
| | 0,15 g | tert.-Butylperoctoat |
| Zulauf 3: | 95,6 g | Butylacetat |
| | 0,39 g | tert.-Butylperoctoat |
| Zulauf 4: | 23 g | Triethanolamin (ca. 20 % bezogen auf Methacrylsäure) |

In einer Rührapparatur mit Rückflusskühler, Innenthermometer und vier separaten Zulaufvorrichtungen wurden bei 85 - 88 °C Zulauf 1 und Zulauf 2 in zwei Stunden zugegeben. Danach wurde das Reaktionsgemisch bei ca. 88 °C 2 h weiter gerührt. Anschließend wurde Zulauf 3 in 30 Minuten zudosiert und 3 Stunden bei 90 °C nachpolymerisiert. Nach dem Abkühlen auf ca. 40 °C wurde das als weißes Pulver ausgefallene Produkt mit Triethanolamin (Zulauf 4) in 1 h bei 40 °C teilneutralisiert. Das Pulver wurde über eine Filternutsche abgesaugt, zweimal mit Aceton gewaschen und bei 40 °C im Vakuum getrocknet.

In analoger Weise wurden die in Tabelle I angegebenen Polymere 1 und 10 bis 20 hergestellt. Das Polymer 17 wurde erst nach der Quatemisierung mit Triethanolamin teilneutralisiert.

### Allgemeine Herstellungsvorschrift A2:

### Beispiel 26: Copolymer aus VP/VI-MAS/VI/MMA/C₁₈-PEG-MA/PETAE

| | | |
|---|---|---|
| Vorlage: | 412 g | Butylacetat |
| Zulauf 1: | 37,5 g | Vinylpyrrolidon |
| | 9,75 g | Vinylimidazol:Methacrylsäure [1:1] |
| | 72,0 g | Vinylimidazol |
| | 30,0 g | Plex-6877 O® (25%ige C₁₈-PEG-MA in Methylmethacrylat) |
| | 0,75 g | Pentaerythrittriallylether |
| Zulauf 2: | 38,2 g | Butylacetat |
| | 0,15 g | tert.-Butylperoctoat |
| Zulauf 3: | 95,6 g | Butylacetat |
| | 0,39 g | tert.-Butylperoctoat |
| Zulauf 4: | 70 g | Methylchlorid |

In einer Druckapparatur mit Rührer, Rückflusskühler, Innenthermometer und vier separaten Zulaufvorrichtungen wurden bei 85 - 88 °C Zulauf 1 und Zulauf 2 in zwei Stunden zugegeben. Das Reaktionsgemisch wurde noch 2 h bei ca. 88 °C gerührt. Danach wurde der Zulauf 3 in 30 Minuten zudosiert und 3 Stunden bei 90 °C nachpolymerisiert. Das als weiße Pulver ausgefallene Produkt wurde mit Methylchlorid (Zulauf 4) in ca. 1 h bei 90 °C quatemisiert. Das Pulver wurde danach über eine Filternutsche abgesaugt, zweimal mit Aceton gewaschen und bei 40 °C im Vakuum getrocknet.

In analoger Weise wurden die in Tabelle I angegebenen Polymere 2 bis 9 und 21 bis 50 hergestellt.

Die in der folgenden Tabelle 1 angegebenen Polymere 3, 4, 7 und 22 bis 50 können auch besonders vorteilhaft nach der im Folgenden beschriebenen Herstellungsvariante B hergestellt werden. Dabei werden in der Regel Polymere mit geringen Restmono- . merengehalten erzielt.

**Tabelle I: VI-MASNP-Fällungspolymerisate**

| | VP [Gew:-%] | VCap [Gew.-%] | VI-MAS [Gew.-%] | MAS [Gew.-%] | VI [Gew.-%] | DMAEMA [Gew.-%] | SMA [Gew.-%] | MMA^{#} [Gew**.-**%] | C₁₈-PEG-MA^{#} [Gew.-%] | ODVE [Gew.-%] | EGDMA [Gew.-%] | PETAE [Gew.-%] | Quatemisierung mit CH₃-Cl | Neutralisierung mit TEA NG % |
|---|---|---|---|---|---|---|---|---|---|---|---|---|---|---|
| 1 | 40 | -- | 10 | 49 | -- | -- | -- | -- | -- | -- | 1,0 | -- | - | 20 |
| 2 | 43,7 | -- | 20 | 35 | -- | -- | -- | -- | -- | -- | 1,3 | -- | ≥70% | -- |
| 3 | 63,7 | -- | 10 | 25 | -- | -- | -- | -- | -- | -- | 1,3 | -- | ≥70% | -- |
| 4 | 73,7 | -- | 10 | 15 | -- | -- | -- | -- | -- | -- | 1,3 | -- | ≥70% | -- |
| 5 | 33,6 | 10 | 20 | 35 | -- | -- | -- | -- | -- | -- | 1,4 | -- | ≥70% | -- |
| 6 | 43,8 | 10 | 20 | 25 | -- | -- | -- | -- | -- | -- | 1,2 | -- | ≥70% | -- |
| 7 | 53,8 | 10 | 15 | 20 | -- | -- | -- | -- | -- | -- | 1,2 | -- | ≥70% | -- |
| 8 | 33,7 | -- | 20 | 35 | -- | -- | -- | 10 | --- | -- | 1,3 | -- | ≥70% | -- |
| 9 | 30 | -- | 20 | 48,8 | -- | -- | -- | -- | -- | -- | 1,2 | -- | ≥70%0% | -- |
| 10 | 30 | -- | 15 | 49 | -- | -- | 5 | -- | -- | -- | 1,0 | -- | -- | 20 |
| 11 | 35 | -- | 10 | 49 | -- | -- | -- | 3,75 | 1,25 | -- | 1,0 | -- | -- | 20 |
| 12 | 40 | -- | 5 | 49 | -- | -- | -- | 3,75 | 1,25 | -- | 1,0 | -- | -- | 20 |
| 13 | 39 | -- | 6 | 48,8 | -- | --- | -- | 5 | -- | -- | 1,2 | -- | -- | 20 |
| 14 | 39 | -- | 10 | 40 | -- | -- | -- | 10 | -- | -- | 1,0 | -- | -- | 20 |
| 15 | 29 | -- | 12 | 38 | -- | -- | -- | 20 | -- | -- | 1,0 | -- | -- | 20 |
| 16 | 39 | -- | 12 | 38 | -- | -- | -- | 7,5 | 2.5 | -- | 1,0 | -- | -- | 20 |
| 17 | 39 | -- | 12 | 40 | -- | -- | -- | -- | -- | 8 | 1,0 | -- | ≥70% | 20 |
| 18 | 39 | -- | 15 | 30 | -- | -- | -- | 11,25 | 3,75 | -- | 1,0 | -- | -- | 20 |
| 19 | 44 | -- | 20 | 33 | -- | -- | 2 | -- | -- | -- | 1,0 | -- | -- | 20 |
| 20 | 40 | -- | 30 | 26 | -- | -- | -- | -- | -- | 3 | 1,0 | -- | -- | 20 |
| 21 | 40 | -- | 30 | 26 | -- | -- | 3 | -- | -- | -- | 1,0 | -- | 75% | -- |
| 22 | 29,5 | -- | 5 | -- | 65 | -- | -- | -- | -- | -- | -- | 0,5 | ≥ 70% | -- |
| 23 | 27 | -- | 5 | -- | 65 | -- | 2,5 | -- | -- | -- | -- | 0,5 | ≥ 70% | -- |
| 24 | 25 | -- | 5 | -- | 60 | -- | -- | 9,5 | -- | -- | -- | 0,5 | ≥ 70% | -- |
| 25 | 25 | -- | 4,5 | -- | 60 | -- | -- | 7,5 | 2,5 | -- | -- | 0,5 | ≥ 70% | -- |
| 26 | 25 | -- | 6,5 | -- | 48 | -- | -- | 15 | 5 | -- | -- | 0,5 | ≥ 70% | -- |
| 27 | 33 | -- | 6,5 | -- | 40 | -- | -- | 15 | 5 | -- | --- | 0,5 | ≥ 70% | -- |
| 28 | 41,5 | -- | 8 | -- | 30 | -- | -- | 15 | 5 | -- | -- | 0,5 | ≥ 70% | -- |
| 29 | 46,5 | --- | 8 | -- | 25 | -- | -- | 15 | 5 | -- | -- | 0,5 | ≥ 70% | -- |
| 30 | 51,5 | -- | 8 | -- | 20 | -- | -- | 15 | 5 | -- | -- | 0,5 | ≥ 70% | -- |
| 31 | 51,5 | -- | 8 | -- | 20 | -- | -- | 20 | -- | -- | -- | 0,5 | ≥ 70% | -- |
| 32 | 70 | -- | 8 | -- | 20 | -- | 1,5 | -- | -- | -- | -- | 0,5 | ≥ 70% | -- |
| 33 | 65 | -- | 10 | -- | 20 | -- | 4,5 | -- | -- | -- | -- | 0,5 | ≥ 70% | -- |
| 34 | 75 | -- | 18 | -- | 15 | -- | -- | -- | -- | 1,5 | -- | 0,5 | ≥ 70% | -- |
| 35 | 66,5 | -- | 8 | -- | 15 | -- | -- | 10 | -- | -- | -- | 0,5 | ≥ 70% | -- |
| 36 | 66,5 | -- | 8 | -- | 15 | -- | -- | 7,5 | 2,5 | -- | -- | 0,5 | ≥ 70% | -- |
| 37 | 56,5 | -- | 8 | -- | 15 | -- | -- | 15 | 5 | -- | -- | 0,5 | ≥ 70% | -- |
| 38 | 17,5 | -- | 8 | -- | 10 | -- | -- | 7,5 | 2,5 | -- | -- | 0,5 | ≥ 80% | -- |
| 40 | 77,5 | -- | 6 | -- | 6 | -- | -- | 7,5 | 2,5 | -- | -- | 0,5 | ≥ 80% | -- |
| 41 | 82,5 | -- | 6 | -- | 6 | -- | -- | 3,75 | 1,25 | -- | -- | 0,5 | ≥ 80% | -- |
| 42 | 85 | -- | 5 | -- | 4,5 | -- | -- | 5 | -- | -- | -- | 0,5 | ≥ 80% | -- |
| 43 | 79,5 | -- | 10 | -- | 10 | -- | -- | -- | -- | -- | -- | 0,5 | ≥ 70% | -- |
| 44 | 69,5 | 10 | 10 | -- | 10 | -- | -- | -- | -- | -- | -- | 0,5 | ≥ 70% | -- |
| 45 | 59,5 | 20 | 10 | -- | 10 | -- | -- | -- | -- | -- | -- | 0,5 | ≥ 70% | -- |
| 46 | 74,5 | -- | 10 | -- | 10 | -- | -- | 3,75 | 1,25 | -- | -- | 0,5 | ≥ 70% | -- |
| 47 | 74,5 | -- | 10 | -- | 10 | -- | -- | -- | -- | 5 | -- | 0,5 | ≥ 70% | -- |
| 48 a) | 65 | -- | 10 | -- | 10 | 10 | 4,5 | -- | -- | -- | -- | 0,5 | ≥ 70% | -- |
| 49 a) | 65 | -- | 15 | -- | -- | 10 | -- | 9,5 | -- | -- | -- | 0,5 | ≥ 70% | -- |
| 50 a) | 59,5 | -- | 20 | -- | -- | 10 | -- | 7,5 | 2,5 | -- | -- | 0,5 | ≥ 70% | -- |

| | | | | | | | | | | | | | | |
|---|---|---|---|---|---|---|---|---|---|---|---|---|---|---|
| a) = nicht erfindungsgemäß VP = N-Vinylpyrrolidon VCap = N-Vinylcaprolactam VI-MAS = Monomerenzusammensetzung aus Vinylimidazol und Methacrylsäure MAS = Methacrylsäure VI = N-Vinylimidazol DMAEMA = N,N-Dimethylaminoethylmethacrylat SMA = Stearylmethacrylat MMA = Methylmethacrylat C₁₈-PEG-MA = mit einem C₁₈-Fettalkohol terminiertes Polyethylenglykolmethacrylat ODVE Octadecylvinylether EGDMA Ethylenglykoldimethacrylat PETAE Pentaerythrittriallylether TEA / NG% Triethanolamin / Neutralisationsgrad *): Das Produkt wurde nach der Quatemisierung mit Triethanolamin teilneutralisiert. # MMA und C₁₈-PEGMA können gemeinsam, z. B. in Form des kommerziellen Produkts Plex-6877 O®, Firma Degussa, Deutschland eingesetzt werden. | | | | | | | | | | | | | | |

### Beispiel 83 (Variante B): Polymerisation von VI/MASNP/PETAE unter Verwendung von zwei Radikalinitiatoren mit unterschiedlicher Zerfallstemperatur

| | | |
|---|---|---|
| Vorlage: | 613 g | Butylacetat |
| | 1 g | Trigonox® 101 (2,5-Dimethyl-2,5-di(tert.-butylperoxy)hexan) |
| Zulauf 1: | 94 g | Vinylpyrrolidon |
| | 102 g | Vinylimidazol |
| | 6,95 g | Methacrylsäure |
| | 1,2 g | Pentaerythrittriallylether |
| Zulauf 2: | 35 g | n-Butylacetat |
| | 0,2 g | tert.-Butylperoctoat |
| Zulauf 3 | 175 g | n-Butylacetat |
| | 1,0 g | tert.-Butylperoctoat |
| Zulauf 4: | 175 g | n-Butylacetat |
| | 1,0 g | tert.-Butylperoctoat |

In einer Apparatur, die mit Rührer, Rückflusszähler, Innenthermometer und vier Zulaufvorrichtungen ausgestattet war, erwärmte man die Vorlage unter Stickstoffatmosphäre auf 90 °C. Die Zuläufe 1 und 2 wurden innerhalb von 3 h zugegeben und bei 90 °C weitere 1,5 h gerührt. Zulauf 3 wurde bei 100 °C in 1 h zugeben, der Reaktionsansatz 1 h bei dieser Temperatur gerührt. Anschließend wurde Zulauf 4 bei 100 °C innerhalb von 1 h zugegeben und dann nochmals 2 h bei 100 °C gerührt. Die Temperatur wurde auf 125 °C erhöht und bei dieser Temperatur noch 2 h weitergerührt. Die erhaltene weiße Suspension wurde anschließend mit 50 g Methylchlorid quatemisiert. Das Produkt wurde filtriert, mit Aceton gewaschen und bei 70 °C im Vakuum getrocknet.

In analoger Weise wurden alle Produkte - der Variante B gemäß Tabelle 2 hergestellt.

### Beispiel 73 (Variante C): Polymerisation von AS/DMAPMAM/PETAE unter Verwendung von zwei Radikalinitiatoren mit unterschiedlicherZerfallstemperatur.

| | | |
|---|---|---|
| Vorlage: | 800 g | Ethylacetat |
| | 1 g | tert.-Butylperoctoat |
| Zulauf 1: | 125 g | Acrylsäure |
| Zulauf 2: | 45 g | DMAPMAM |
| | 1,6 g | Pentaerythrittriallylether |
| Zulauf 3 | 80 g | Ethylacetat |
| | 0,4 g | Lauroylperoxid |
| Zulauf 4: | 200 g | Ethylacetat |
| | 0,4 g | Lauroylperoxid |

In einer Apparatur, die mit Rührer, Rückflusszähler, Innenthermometer und vier Zulaufvorrichtungen ausgestattet war, erwärmte man die Vorlage unter Stickstoffatmosphäre und Rühren auf 75 °C. Die Zuläufe 1, 2 und 3 wurden innerhalb von 3 h zugegeben und bei 75 °C weitere 2 h gerührt. Zulauf 4 wurde in 1 h bei 80 °C zugeben und dann noch 1 h weitergerührt. Die Temperatur wurde auf 100 °C erhöht und bei dieser Temperatur noch weitere 3 h gerührt. Die erhaltene weiße Suspension wurde anschließend mit 40 g Methylchlorid quatemisiert. Das Produkt wurde filtriert, mit Aceton gewaschen und bei 70 °C im Vakuum getrocknet.

In analoger Weise wurden alle Produkte der Variante C gemäß Tabelle 2 hergestellt.

### Beispiel 52 (Variante D): Polymerisation von AS/DMAPMAM/SMA/PETAE unter Verwendung von zwei Radikalinitiatoren mit unterschiedlicher Zerfallstemperatur

| | | |
|---|---|---|
| Vorlage: | 670 g | Ethylacetat/Cyclohexan (65:35) |
| | 50 g | Zulauf 1 |
| | 14 g | Zulauf 2 |
| | 1,5 g | Pentaerythrittriallylether |
| | 1,5 g | tert.-Butylperoctoat |
| Zulauf 1: | 142,5 g | Acrylsäure |
| | 3 g | Stearylmethacrylat |
| | 3 g | Dimethylaminopropylmethacrylamid |
| | 100 g | Ethylacetat/Cyclohexan (65:35) |
| | 4,3 g | wasserfreies K₂CO₃ |
| Zulauf 2: | 70 g | Ethylacetat/Cyclohexan (65:35) |
| | 0,35 g | Trigonox® EHP-C75 (75%ig) |
| Zulauf 3 | 70 g | Ethylacetat/Cyclohexan (65:35) |
| | 1,0 g | Trigonox® EHP-C75 (75%ig) |

In einer Apparatur, die mit Rührer, Rückflusszähler, Innenthermometer und drei Zulaufvorrichtungen ausgestattet war, erwärmte man die Vorlage unter Stickstoffatmosphäre und Rühren auf 50 °C. Der Zulauf 1 wurde innerhalb von 1,5 h und der Zulauf 2 innerhalb von 2 h zugegeben und der Ansatz bei 60 °C noch weitere 2 h gerührt. Zulauf 3 wurde in 1 h bei 60 °C zugegeben und dann noch 2 h bei 70 °C gerührt. Die Temperatur wurde auf 100 °C erhöht und bei der Temperatur noch 3 h weitergerührt. Die erhaltene weiße Suspension wurde filtriert, mit Aceton gewaschen und bei 70 °C im Vakuum getrocknet.

In analoger Weise wurden alle Produkte der Variante D gemäß Tabelle 2 hergestellt.

**Tabelle 2:**

| Bsp. | VP^{#} | MAS^{#} | AS^{#} | VI^{#} | DMAPMAM^{#} | SMA^{#} | n-BA^{#} | EMA^{#} | PLEX-O^{#} | EGDMA^{#} | PETAE^{#} | Herstellungsvariante |
|---|---|---|---|---|---|---|---|---|---|---|---|---|
| 51 | -- | 90 | -- | 4 | -- | 5 | -- | -- | -- | 1,0 | -- | C |
| 52 a) | -- | -- | 95 | -- | 2 | 2 | -- | -- | -- | -- | 1,0 | D |
| 53 a) | -- | 85 | -- | 10 | -- | 3,5 | -- | -- | -- | 1,5 | -- | C |
| 54 a) | -- | -- | 92 | -- | 4 | 2,5 | -- | -- | -- | -- | 1,5 | D |
| 55 a) | -- | -- | 85 | -- | 12 | 1,8 | -- | -- | -- | -- | 1,2 | C |
| 56 a) | -- | 85 | -- | 5 | 5 | 3,8 | -- | -- | -- | 1,2 | -- | C |
| 57 a) | -- | 78 | -- | 10 | 7 | 3,8 | -- | -- | -- | 1,2 | -- | B |
| 58 a) | -- | -- | 75 | -- | 10 | -- | -- | 13,5 | -- | -- | 1,5 | C |
| 59 a) | -- | 75 | -- | -- | 23,5 | -- | -- | -- | -- | 1,5 | -- | C |
| 60 a) | -- | 70 | -- | -- | 23,5 | -- | -- | -- | 5 | 1,5 | -- | C |
| 61 a) | -- | 45 | -- | -- | 48,5 | -- | -- | -- | 5 | 1,5 | -- | C |
| 62 a) | -- | 50 | -- | 25 | 23,8 | -- | -- | -- | -- | 1,2 | -- | C |
| 63 a) | -- | 50 | -- | 25 | 20 | 3,8 | -- | -- | -- | 1,2 | -- | C |
| 64 a) | -- | 45 | -- | -- | 38,5 | -- | 15 | -- | -- | 1,5 | -- | C |
| 65 a) | -- | 45 | -- | -- | 38,5 | -- | 10 | -- | 5 | 1,5 | -- | C |
| 66 a) | -- | 45 | -- | -- | 33,5 | -- | 15 | -- | 5 | 1,5 | -- | C |
| 67 a) | -- | 45 | -- | -- | 33,5 | -- | 25 | -- | -- | 1,5 | -- | C |
| 68 a) | -- | 45 | -- | -- | 28,5 | -- | 25 | -- | -- | 1,5 | -- | C |
| 69 a) | -- | 45 | -- | -- | 28 | -- | 25 | -- | -- | 2 | -- | C |
| 70 a) | -- | 45 | -- | -- | 23,5 | -- | 30 | -- | -- | 1,5 | -- | C |
| 71 a) | -- | 40 | -- | -- | 23,5 | -- | 35 | -- | -- | 1,5 | -- | C |
| 72 a) | -- | 70 | -- | -- | 28,8 | -- | -- | --- | -- | 1,2 | -- | C |
| 73 a) | -- | - | 70 | -- | 28,8 | -- | -- | -- | -- | -- | 1,2 | C |
| 74 a) | 25 | 70 | -- | -- | 3,8 | -- | -- | -- | -- | 1,2 | -- | B |
| 75 a) | 25 | - | 70 | -- | 3,8 | -- | -- | -- | --- | 1,2 | -- | B |
| 76 a) | 25 | 50 | -- | -- | 23,8 | -- | -- | -- | -- | 1,2 | -- | C |
| 77 a) | 25 | - | 50 | -- | 23,8 | -- | -- | -- | -- | -- | 1,2 | C |
| 78 a) | 45 | 50 | -- | -- | 3,8 | -- | -- | -- | -- | 1,2 | -- | C |
| 79 a) | 45 | | 50 | -- | 3,8 | -- | -- | -- | -- | -- | 1,2 | C |
| 80 a) | 45 | 30 | -- | -- | 23,8 | -- | -- | -- | -- | 1,2 | -- | C |
| 81 a) | 45 | - | 30 | - | 23,8 | -- | -- | -- | -- | 1,2 | -- | C |
| 82 | 61 | 3,3 | -- | 35 | -- | -- | -- | -- | -- | -- | 0,7 | B |
| 83 | 46 | 3,3 | -- | 50 | -- | -- | -- | -- | --- | -- | 0,7 | B |
| 84 | 31 | 3,3 | -- | 65 | -- | -- | -- | -- | -- | -- | 0,7 | B |
| 85 | 16 | 3,3 | -- | 80 | -- | -- | -- | -- | -- | -- | 0,7 | B |
| 86 | -- | 4,5 | -- | 85 | -- | -- | -- | 10 | -- | -- | 0,5 | B |
| 87 | -- | 5,5 | -- | 94 | -- | -- | -- | - | -- | -- | 0,5 | B |
| 88 | -- | 5,5 | -- | 90 | -- | 4 | -- | 4 | -- | -- | 0,5 | B |
| 89 | -- | 3,3 | -- | 93 | -- | 3 | -- | 3 | -- | -- | 0,7 | B |
| 90 | -- | 2,3 | -- | 95 | -- | 2 | -- | 2 | -- | -- | 0,7 | B |

| | | | | | | | | | | | | |
|---|---|---|---|---|---|---|---|---|---|---|---|---|
| a) = nicht erfindungsgemäß Bsp. Beispiel # Die Mengenangaben sind in Gew.%, bezogen auf die zur Polymerisation eingesetzten ungesättigten Verbindungen VP Vinylpyrrolidon MAS Methacrylsäure AS Acrylsäure VI Vinylimidazol DMAPMAM Dimethylaminopropylmethacrylamid SMA Stearylmethacrylat EGDMA Ethylenglykoldimethacrylat PETAE Pentaerythrittriallylether EMA Ethylmethacrylat n-BA n-Butylacrylat PLEX-O Plex® 6877-0 = Methacrylsäureester eines mit 25 Mol Ethylenoxid alkoxilierten C₁₆-C₁₈-Fettalkohols als 25%ige Lösung in Methylmethacrylat | | | | | | | | | | | | |

## Patentansprüche

1. Ampholytisches Copolymer A), erhättlich durch radikalische Copolymerisation nach der Methode der Fällungspolymerisation, das Verbindungen, bestehend aus
a) 2 bis 96 Gew.-% Acrylsäure und/oder Methacrylsäure,
b) 2 bis 96 Gew.-% wenigstens einer N-Vinylimidazol-Verbindung der allgemeinen Formel (II) 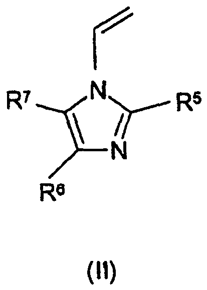 worin R⁶ bis R⁷ unabhängig voneinander für Wasserstoff, C₁-C₄-Alkyl oder
Phenyl stehen, oder die durch Protonierung oder Quaternisierung der N- Vinylmidazol- Verbindungen erhält lichen Verbindung,
c) 0,05 bis 5 Gew.-% Ethylenglykoldi(meth)acrylat und/oder Pentaerythrittriallylether,
d) 0 bis 95 Gew.-% Vinylpyrrolidon und/oder Vinylcaprolactam,
e) 0 bis 40 Gew.-% wenigstens einer Verbindung, die ausgewählt ist unter C₈-C₂₂-(Meth)acrylaten, C₈-C₂₂-Alkylvinylethem, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestem und Mischungen davon,
g) 0 bis 40 Gew.-% wenigstens eines Monomers, das ausgewählt ist unter Methyl(meth)acrylat, Ethyl(meth)acrylat, n-Butyl(meth)acrylat und Mischungen davon,
einpolymerisiert enthält,
wobei zur Copolymerisation Monomere mit ionogenen oder ionischen Gruppen in solchen Mengen eingesetzt werden, dass das Copolymer A) einen molaren Überschuss an anionogenen/anionischen Gruppen gegenüber kationogenen/- kationischen Gruppen oder einen molaren Überschuss an kationogenen/kationischen Gruppen gegenüber anionogenen/anionischen Gruppen von wenigstens 2,5 : 1, aufweist.

2. Ampholytisches Copolymer nach Anspruch 1, wobei wenigstens ein Teil der Verbindungen a) und b) in Form eines Monomerenpaares eingesetzt wird, wobei das molare Verhältnis von anionogenen Gruppen der Komponente a) zu kationogenen Gruppen der Komponente b) 1:1 beträgt und wobei die im Unterschuss eingesetzte Komponente a) oder b) vollständig als Komponente des Monomerenpaares eingesetzt wird.

3. Ampholytisches Copolymer A) nach einem der vorhergehenden Ansprüche, wobei Monomer b) 1-Vinylimidazol ist.

4. Copolymer nach einem der Ansprüche 1 bis 3, erhältlich durch radikalische Copolymerisation von
- wenigstens 2 Gew.-%, bezogen auf das Gesamtgewicht der zur Polymerisation eingesetzten Monomere, wenigstens eines Monomerenpaars aus N-Vinylimidazol und Acrylsäure und/oder Methacrylsäure,
- 3 bis 70 Gew.-% Vinylimidazol-Verbindungen,
- 0,1 bis 2 Gew.-% Ethylenglykoldi(meth)acrylat und/oder Pentaerythrittriallylether,
- 0 bis 95 Gew.-% Vinylpyrrolidon und/oder Vinylcaprolactam d),
- 0 bis 40 Gew.-% wenigstens eines weiteren Monomers, das ausgewählt ist unter Methyl(meth)acrylat, Ethyl(meth)acrylat, n-Butyl(meth)acrylat, C₈-C₂₂-Meth)acrylaten, C₈-C₂₂-Alkylvinylethern, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestern und Mischungen davon.

5. Copolymer nach einem der Ansprüche 1 bis 3, erhältlich durch radikalische Copolymerisation von
- wenigstens 5 Gew.-%, bezogen auf das Gesamtgewicht der zur Polymerisation eingesetzten Monomere, wenigstens eines Monomerenpaars aus N-Vinylimidazol und Acrylsäure und/oder Methacrylsäure,
- 3 bis 70 Gew.-% wenigstens einer Vinylimidazol-Verbindung,
- 0,1 bis 2 Gew.% Ethylenglykoldi(meth)acrylat und/oder Pentaerythrittriallylether,
- 20 bis 85 Gew.-% Vinylpyrrolidon und/oder Vinylcaprolactam d),
- 1 bis 20 Gew.-% wenigstens eines weiteren Monomers, das ausgewählt ist unter C₈-C₃₀-(Meth)acrylaten, mit C₈-C₃₀-Alkylgruppen terminierten Polyether(meth)acrylaten und Mischungen davon, insbesondere unter Stearylmethacrylat, mit C₁₈-C₂₂-Alkylgruppen terminierten Polyethylenglycol(meth)acrylaten und Mischungen davon.

6. Verfahren zur Herstellung eines ampholytisches Copolymer A), wie in einem der Ansprüche 1 bis 5 definiert, durch radikalische Copolymerisation nach der Methode der Fällungspolymerisation.

7. Verfahren nach Anspruch 6, wobei die Polymerisation in einem wasserfreien, aprotischen Lösungsmittel oder Lösungsmittelgemisch, vorzugsweise in Ethylacetat und/oder n-Butylacetat, erfolgt

8. Verfahren nach einem der Ansprüche 6 oder 7, bei dem zur Copolymerisation wenigstens zwei Initiatoren eingesetzt werden, deren Zerfallstemperaturen um wenigstens 10 °C, vorzugsweise um wenigstens 15 °C, voneinander verschieden sind.

9. Verfahren nach Anspruch 8, wobei die Copolymerisation bis zum Abschluss der Fällung des Copolymers bei einer Temperatur größer oder gleich der niedrigeren Zerfallstemperatur und kleiner der höheren Zerfallstemperatur erfolgt und nach der Fällung eine weitere Umsetzung bei einer Temperatur größer oder gleich der höheren Zerfallstemperatur erfolgt.

10. Verfahren nach einem der Ansprüche 6 bis 9, umfassend eine erste Polymerisationsphase bei einer ersten Polymerisationstemperatur und eine zweite Polymerisationsphase bei einer zweiten Polymerisationstemperatur oberhalb der ersten Polymerisationstemperatur, wobei zur Polymerisation wenigstens zwei Initiatoren eingesetzt werden, deren Halbwertszeiten bei der ersten Polymerisationstemperatur sich so unterscheiden, dass wenigstens einer dieser Initiatoren während der ersten Polymerisationsphase in Radikale zerfällt und wenigstens einer dieser Initiatoren während der ersten Polymerisationsphase im Wesentlichen nicht in Radikale zerfällt und während der zweiten Polymerisationsphase in Radikale zerfällt.

11. Kosmetisches oder pharmazeutisches Mittel, enthaltend
A) wenigstens ein ampholytisches Copolymer wie in einem der Ansprüche 1 bis 5 definiert,
B) wenigstens einen kosmetisch oder pharmazeutisch akzeptablen Wirk- oder Effektstoff, und
C) gegebenenfalls wenigstens einen weiteren, von B) verschiedenen kosmetisch oder pharmazeutisch akzeptablen Wirk- oder Hilfsstoff.

## Claims

1. An ampholytic copolymer A) obtainable by free radical copolymerization in accordance with the method of precipitation polymerization, which comprises, in copolymerized form, compounds consisting of
a) 2 to 96% by weight of acrylic acid and/or methacrylic acid,
b) 2 to 96% by weight of at least one N-vinylimidazole compound of the general formula (II) 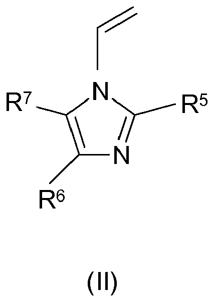 in which R⁵ to R⁷, independently of one another, are hydrogen, C₁-C₄-alkyl or phenyl, or the compounds obtainable by protonation or quaternization of the N-vinylimidazole compounds,
c) 0.05 to 5% by weight of ethylene glycol di(meth)acrylate and/or pentaerythritol triallyl ether,
d) 0 to 95% by weight of vinylpyrrolidone and/or vinylcaprolactam,
e) 0 to 40% by weight of at least one compound which is chosen from C₈-C₂₂-(meth)acrylates, C₈-C₂₂-alkyl vinyl ethers, polyether (meth)acrylates terminated with C₈-C₂₂-alkyl groups, allyl alcohol alkoxylates terminated with C₈-C₂₂-alkyl groups, C₈-C₂₂-carboxylic acid vinyl esters and mixtures thereof,
g) 0 to 40% by weight of at least one monomer which is chosen from methyl (meth)acrylate, ethyl (meth)acrylate, n-butyl (meth)acrylate and mixtures thereof,
where, for the copolymerization, monomers with ionogenic or ionic groups are used in amounts such that the copolymer A) has a molar excess of anionogenic/anionic groups compared with cationogenic/cationic groups or a molar excess of cationogenic/cationic groups compared with anionogenic/anionic groups of at least 2.5:1.

2. The ampholytic copolymer according to claim 1, where at least part of the compounds a) and b) is used in the form of a monomer pair, where the molar ratio of anionogenic groups of component a) to cationogenic groups of component b) is 1:1 and where the component a) or b) used in deficit is used completely as component of the monomer pair.

3. The ampholytic copolymer A) according to one of the preceding claims, where monomer b) is 1-vinylimidazole.

4. The copolymer according to one of claims 1 to 3, obtainable by free-radical copolymerization of
- at least 2% by weight, based on the total weight of the monomers used for the polymerization, of at least one monomer pair of N-vinylimidazole and acrylic acid and/or methacrylic acid,
- 3 to 70% by weight of vinylimidazole compounds,
- 0.1 to 2% by weight of ethylene glycol di(meth)acrylate and/or pentaerythritol triallyl ether,
- 0 to 95% by weight of vinylpyrrolidone and/or vinylcaprolactam d),
- 0 to 40% by weight of at least one further monomer which is chosen from methyl (meth)acrylate, ethyl (meth)acrylate, n-butyl (meth)acrylate, C₈-C₂₂-(meth)acrylates, C₈-C₂₂-alkyl vinyl ethers, polyether (meth)acrylates terminated with C₈-C₂₂-alkyl groups, allyl alcohol alkoxylates terminated with C₈-C₂₂-alkyl groups, C₈-C₂₂-carboxylic acid vinyl esters and mixtures thereof.

5. The copolymer according to one of claims 1 to 3, obtainable by free-radical copolymerization of
- at least 5% by weight, based on the total weight of the monomers used for the polymerization, of at least one monomer pair of N-vinylimidazole and acrylic acid and/or methacrylic acid,
- 3 to 70% by weight of at least one vinylimidazole compound,
- 0.1 to 2% by weight of ethylene glycol di(meth)acrylate and/or pentaerythritol triallyl ether,
- 20 to 85% by weight of vinylpyrrolidone and/or vinylcaprolactam d),
- 1 to 20% by weight of at least one further monomer which is chosen from C₈-C₃₀-(meth)acrylates, polyether (meth)acrylates terminated with C₈-C₃₀-alkyl groups, and mixtures thereof, in particular from stearyl methacrylate, polyethylene glycol (meth)acrylates terminated with C₁₈-C₂₂-alkyl groups, and mixtures thereof.

6. A process for the preparation of an ampholytic copolymer A) as defined in one of claims 1 to 5 by free-radical copolymerization in accordance with the method of precipitation polymerization.

7. The process according to claim 6, where the polymerization takes place in an anhydrous, aprotic solvent or solvent mixture, preferably in ethyl acetate and/or n-butyl acetate.

8. The process according to one of claims 6 and 7, in which, for the copolymerization, at least two initiators are used whose decomposition temperatures are different from one another by at least 10°C, preferably by at least 15°C.

9. The process according to claim 8, where the copolymerization takes place until the conclusion of the precipitation of the copolymer at a temperature greater than or equal to the lower decomposition temperature and less than the higher decomposition temperature and, after the precipitation, a further reaction takes place at a temperature greater than or equal to the higher decomposition temperature.

10. The process according to one of claims 6 to 9, comprising a first polymerization phase at a first polymerization temperature and a second polymerization phase at a second polymerization temperature above the first polymerization temperature, where, for the polymerization, at least two initiators are used whose half-lives at the first polymerization temperature differ in such a way that at least one of these initiators decomposes into free radicals during the first polymerization phase and at least one of these initiators essentially does not decompose into free radicals during the first polymerization phase and decomposes into free radicals during the second polymerization phase.

11. A cosmetic or pharmaceutical composition comprising
A) at least one ampholytic copolymer as defined in one of claims 1 to 5,
B) at least one cosmetically or pharmaceutically acceptable active substance or effect substance, and
C) optionally at least one further cosmetically or pharmaceutically acceptable active substance or auxiliary different from B).

## Revendications

1. Copolymère ampholyte A), pouvant être obtenu par copolymérisation radicalaire selon la méthode de la polymérisation par précipitation, qui contient, incorporés par polymérisation, des composés consistant en
a) 2 à 96% en poids d'acide acrylique et/ou d'acide méthacrylique,
b) 2 à 96 % en poids d'au moins un composé N-vinylimidazole de formule générale (II) 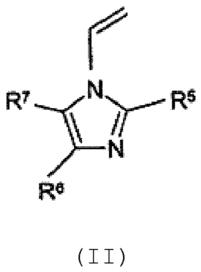 dans laquelle R⁵ à R⁷ représentent chacun indépendamment un atome d'hydrogène, un groupe alkyle en C₁-C₄ ou phényle, ou les composés pouvant être obtenus par protonation ou quaternisation des composés N-vinylimidazole,
c) 0,05 à 5 % en poids de di(méth)acrylate d'éthylèneglycol et/ou d'éther triallylique de pentaérythritol,
d) 0 à 95 % en poids de vinylpyrrolidone et/ou vinylcaprolactame,
e) 0 à 40 % en poids d'au moins un composé qui est choisi parmi des (méth)acrylates en C₈-C₂₂, alkyl(C₈-C₂₂)vinyléthers, polyéther (méth) acrylates terminés par des groupes alkyle en C₈-C₂₂, produits d'alcoxylation d'alcool allylique terminés par des groupes alkyle en C₈-C₂₂, esters vinyliques d'acides carboxyliques en C₈-C₂₂ et des mélanges de ceux-ci,
g) 0 à 40 % en poids d'au moins un monomère qui est choisi parmi le (méth)acrylate de méthyle, le (méth)acrylate d'éthyle, le (méth)acrylate de n-butyle et des mélanges de ceux-ci,
en utilisant dans la copolymérisation des monomères à groupes ioniques ou ionogènes en quantités telles que le copolymère A) présente un excès molaire de groupes anioniques/anionogènes par rapport aux groupes cationiques/cationogènes ou un excès molaire de groupes cationiques/cationogènes par rapport aux groupes anioniques/anionogènes d'au moins 2,5:1.

2. Copolymère ampholyte selon la revendication 1, dans lequel on utilise au moins une partie des composés a) et b) sous forme d'une paire de monomères, le rapport molaire des groupes anionogènes du composant a) aux groupes cationogènes du composant b) valant 1:1 et dans lequel le composant a) ou b) utilisé en quantité insuffisante est utilisé en totalité comme composant de la paire de monomères.

3. Copolymère ampholyte A) selon l'une quelconque des revendications précédentes, dans lequel le monomère b) est le 1-vinylimidazole.

4. Copolymère selon l'une quelconque des revendications 1 à 3, pouvant être obtenu par copolymérisation radicalaire de
- au moins 2 % en poids, par rapport au poids total des monomères utilisés dans la polymérisation, d'au moins une paire de monomères constituée de N-vinylimidazole et acide acrylique et/ou acide méthacrylique,
- 3 à 70 % en poids de composés vinylimidazole,
- 0,1 à 2 % en poids de di(méth)acrylate d'éthylèneglycol et/ou d'éther triallylique de pentaérythritol,
- 0 à 95 % en poids de vinylpyrrolidone et/ou vinylcaprolactame d),
- 0 à 40 % en poids d'au moins un autre monomère qui est choisi parmi le (méth)acrylate de méthyle, le (méth)acrylate d'éthyle, le (méth)acrylate de n-butyle, des (méth)acrylates en C₈-C₂₂, des alkyl(C₈-C₂₂)vinyléthers, des polyéther(méth)-acrylates terminés par des groupes alkyle en C₈-C₂₂, des alcoxylates d'alcool allylique terminés par des groupes alkyle en C₈-C₂₂, des esters vinyliques d'acides carboxyliques en C₈-C₂₂ et des mélanges de ceux-ci.

5. Copolymère selon l'une quelconque des revendications 1 à 3, pouvant être obtenu par copolymérisation radicalaire de
- au moins 5 % en poids, par rapport au poids total des monomères utilisés dans la polymérisation, d'au moins une paire de monomères constituée de N-vinylimidazole et acide acrylique et/ou acide méthacrylique,
- 3 à 70 % en poids d'au moins un composé vinylimidazole,
- 0,1 à 2 % en poids de di(méth)acrylate d'éthylèneglycol et/ou d'éther triallylique de pentaérythritol,
- 20 à 85 % en poids de vinylpyrrolidone et/ou vinylcaprolactame d),
- 1 à 20 % en poids d'au moins un autre monomère qui est choisi parmi des (méth)acrylates en C₈-C₃₀, des polyéther(méth)acrylates terminés par des groupes alkyle en C₈-C₃₀ et des mélanges de ceux-ci, en particulier parmi le méthacrylate de stéaryle, des (méth)acrylates de polyéthylèneglycol terminés par des groupes alkyle en C₁₈-C₂₂ et des mélanges de ceux-ci.

6. Procédé pour la préparation d'un copolymère ampholyte A), tel que défini dans l'une quelconque des revendications 1 à 5, par copolymérisation radicalaire selon la méthode de la polymérisation par précipitation.

7. Procédé selon la revendication 6, dans lequel la polymérisation s'effectue dans un solvant ou mélange de solvants aprotique(s) anhydre, de préférence dans de l'acétate d'éthyle et/ou de l'acétate de n-butyle.

8. Procédé selon la revendication 6 ou 7, dans lequel on utilise dans la copolymérisation au moins deux amorceurs, dont les températures de décomposition diffèrent entre elles d'au moins 10 °C, de préférence d'au moins 15 °C.

9. Procédé selon la revendication 8, dans lequel la polymérisation s'effectue jusqu'à la fin de la précipitation du copolymère à une température supérieure ou égale à la plus basse température de décomposition et inférieure à la température de décomposition la plus élevée et après la précipitation une nouvelle réaction s'effectue à une température supérieure ou égale à la température de décomposition la plus élevée.

10. Procédé selon l'une quelconque des revendications 6 à 9, comprenant une première phase de polymérisation à une première température de polymérisation et une deuxième phase de polymérisation à une deuxième température de polymérisation supérieure à la première température de polymérisation, dans lequel on utilise dans la polymérisation au moins deux amorceurs, dont les temps de demi-vie à la première température de polymérisation diffèrent de manière qu'au moins l'un de ces amorceurs se décompose en radicaux pendant la première phase de polymérisation et au moins l'un de ces amorceurs essentiellement ne se décompose pas en radicaux pendant la première phase de polymérisation et se décompose en radicaux pendant la deuxième phase de polymérisation.

11. Composition cosmétique ou pharmaceutique, contenant
A) au moins un copolymère ampholyte tel que défini dans l'une quelconque des revendications 1 à 5,
B) au moins une substance active ou à effet cosmétiquement ou pharmaceutiquement acceptable, et
C) éventuellement au moins un autre actif ou adjuvant cosmétiquement ou pharmaceutiquement acceptable, différent de B).
